# EUROPÄISCHE PATENTSCHRIFT

(11) **EP 3 423 432 B1**
(45) Veröffentlichungstag und Bekanntmachung des Hinweises auf die Patenterteilung: **17.02.2021**
(21) Anmeldenummer: 17708721.0
(22) Anmeldetag: 27.02.2017
(51) Int. Cl.: C07C 273/10, B01D 11/00, B01J 10/00, B01J 8/04

(54) **INTEGRIERTE SYNTHESE VON AMMONIAK UND HARNSTOFF**
INTEGRATED SYNTHESIS OF AMMONIA AND UREA
SYNTHÈSE INTÉGRÉE DE L'AMMONIAC ET DE L'URÉE

(30) Priorität: 29.02.2016 DE 102016203277
(43) Veröffentlichungstag der Anmeldung: 09.01.2019
(73) Patentinhaber: thyssenkrupp Industrial Solutions AG, 45143 Essen (DE); thyssenkrupp AG, 45143 Essen (DE)
(72) Erfinder: JOHANNING, Joachim, 46045 Oberhausen (DE); MAKHYNYA, Yevgeny, 45481 Mülheim an der Ruhr (DE)
(74) Vertreter: thyssenkrupp Intellectual Property GmbH
(86) Internationale Anmeldenummer: PCT/EP2017/054523
(87) Internationale Veröffentlichungsnummer: WO 2017/148870

(56) Entgegenhaltungen:
- US-A- 4 869 887
- US-B1- 7 642 377

## Beschreibung

Die Erfindung betrifft die integrierte Synthese von NH₃ aus Synthesegas unter Abtrennung von CO₂ sowie die Synthese von Harnstoff aus dem NH₃ und dem abgetrennten CO₂.

Die großtechnische Herstellung von Harnstoff beruht derzeit praktisch ausschließlich auf der Hochdrucksynthese von Ammoniak (NH₃) und Kohlendioxid (CO₂) bei ca. 150 bar und ca. 180 °C. Beide Einsatzstoffe kommen in der Regel aus einer NH₃-Anlage, die meist in enger Nachbarschaft zu der betreffenden Harnstoff-Anlage steht.

In der Vergangenheit wurden etliche Konzepte für eine prozessseitige Integration von NH₃-Anlage einerseits und Harnstoff-Anlage andererseits vorgeschlagen, die ältesten wurden sogar Anfang des 20. Jahrhunderts veröffentlicht. Die meisten Konzepte beruhen auf der Abtrennung des CO₂ aus dem Rohsynthesegas der NH₃-Anlage mit prozesseigenem NH₃ oder einem hochkonzentrierten NH₃/H₂O-Gemisch und weiterem Transfer des Produktes - überwiegend als Ammoniumcarbamat - zum Harnstoff-Reaktor. Ziel dieser prozessseitigen Integration war, sowohl den Energiebedarf der CO₂-Verdichtung zu eliminieren als auch den apparativen Aufwand für die Anlage durch den faktischen Wegfall der CO₂-Kompression und der CO₂-Wäsche insgesamt zu reduzieren.

Ist Wasser in der Lösung in nennenswerten Mengenanteilen vorhanden, so können neben Carbamat- auch Hydrogencarbonat- und Carbonat-Ionen gebildet werden. Ammoniumcarbamat ist dabei gleichzeitig das Edukt in der Reaktion zum Harnstoff, die anderen Produkte nicht. Die Carbonate können unter anderem aus Ammoniumcarbamat durch Reaktion mit Wasser entstehen und sind in Hinsicht auf maximale Harnstoffausbeute unerwünscht. Die bei der CO₂-Abtrennnung entstehenden Produkte können nur dann mit vergleichsweise geringem Aufwand in die Harnstoff-Synthese eingebracht werden, wenn eine flüssige Phase - das Reaktionsmedium - vorhanden ist.

DE 1 668 547 offenbart ein Verfahren zur Herstellung von NH₃ und Harnstoff, das dadurch gekennzeichnet ist, dass man CO₂, N₂ und H₂ enthaltendes NH₃-Synthesegas in eine erste Zone leitet, die unter solchen Bedingungen gehalten wird, dass CO₂ aus dem Synthesegas in einer ammoniakhaltigen Flüssigkeit abgetrennt und ein Ammoniumcarbamat enthaltendes Kondensat erhalten wird, und dass man das restliche Ammoniaksynthesegas in eine Ammoniaksynthesezone leitet und das Kondensat in eine zweite Zone leitet, die unter zur Herstellung von Harnstoff aus dem Kondensat geeigneten Bedingungen gehalten wird. Das ist ein typisches Beispiel eines vollintegrierten Verfahrens, bei dem keine direkte CO₂-Abtrennung stattfindet.

DE 25 26 353 betrifft ein integriertes Verfahren zur Herstellung von Harnstoff und NH₃. Dabei erfolgt eine Entfernung des NH₃ aus dem Krcislaufgas durch Wasscrwäschc. CO₂ wird aus dem rohen Synthesegas durch NH₃/H₂O-Wäsche ausgewaschen.

DE 26 13 102 C2 offenbart ein Verfahren zur gleichzeitigen Herstellung von NH₃ und Harnstoff, bei dem ein beim Reformieren von Kohlenwasserstoffen und anschließender CO-Umwandlung erhaltenes Gasgemisch aus CO₂, N₂ und H₂ einer Absorption des Kohlendioxids mittels Ammoniaklösung, die bei einer Absorption des Ammoniaks aus der Ammoniaksynthese mittels Wasser anfällt, zugeführt und die so gebildete Ammoniumcarbamatlösung in die Harnstoffsynthese geführt wird.

US 4,138,434 betrifft ein Prozesskonzept mit Integration von NH₃- und Harnstoff-Synthese. Dabei erfolgt eine zweistufige Auswaschung von CO₂ aus dem rohen Synthesegas mit wässrigem Ammoniak unter Bildung von Ammoniumcarbamat. Restliches NH₃ wird mit rohem Synthesegas ausgetrieben. Es erfolgt ein thermisches Strippen, wobei für das Kompensieren von Druckverlusten ein Ejektor eingesetzt wird.

Bisher wurde allerdings noch keines dieser Konzepte großtechnisch realisiert. Gründe hierfür sind nicht bekannt geworden. Es liegt die Vermutung nahe, dass die Inbetriebnahme der beiden integrierten Anlagen (NH₃ und Harnstoff) gleichzeitig erfolgen muss und dadurch deutlich komplizierter wird und ein - mit der konventionellen Prozessführung möglicher - separater Betrieb von NH₃-Anlage oder Harnstoff-Anlage höchstwahrscheinlich nicht mehr realisierbar ist.

In allen bisher veröffentlichen Konzepten wird die Abtrennung des CO₂ aus dem Rohsynthesegas unter Kühlung durchgeführt, um den Temperaturanstieg und den NH₃-Verlust in der Waschkolonne zu begrenzen. Dabei geht die wertvolle Reaktionsenthalpie der Carbamat-Bildung als Niedertemperaturwärme verloren. Weiterhin fehlt diese Wärme dann bei der späteren Umwandlung des Carbamates in Harnstoff komplett und muss von extern eingebracht werden.

Über die möglichen Konzepte zur Wärmeintegration und über die Quellen der externen Wärme sowie über die Konsequenzen für die Wirtschaftlichkeit des Prozesses ist nichts bekannt. Zudem kommen je nach Integrationskonzept noch rein chemische Probleme wie z.B. zusätzlicher beträchtlicher Wasscrcintrag in die Harnstoffsynthese oder die Verringerung der Partialdrücke der Reaktionspartner, wodurch das Gleichgewicht der Harnstoff-Bildungsreaktion ungünstig beeinflusst wird.

Die Harnstoffsynthese aus NH₃ und CO₂ ist insgesamt exotherm. Sie besteht aus der relativ stark exothermen und verhältnismäßig schnellen Reaktion der Edukte zu Ammoniumcarbamat sowie der deutlich langsameren und endothermen Weiterreaktion zu Harnstoff und Wasser. Eine gute Energieeffizienz des Gesamtverfahrens lässt sich nur erreichen, wenn die freiwerdende Reaktionswärme der Carbamatbildungsreaktion für die Harnstoffbildung zumindest anteilig genutzt wird.

Die Speicherung dagegen impliziert die Abkühlung des Reaktionsgemisches und resultiert in zusätzlichen Energieaufwänden. Auf diesem Weg kann eine günstige Gesamtlösung nur durch ausgeklügelte Wärmeintegration im gesamten Anlagen-Komplex realisiert werden.

Bei einer CO₂-Wäsche mit NH₃- bzw. NH₃/H₂O-Gemischen wird bei der Carbamatbildung viel Wärme freigesetzt. Wasser könnte viel effektiver als NH₃ die Wärme aufnehmen, ist aber nur wenig oder überhaupt nicht vorhanden. Für die Rückhaltung des NH₃ muss daher gekühlt werden. Bei den bisher vorgeschlagenen Konzepten muss diese Wärme aber größtenteils über Kühlwasser ungenutzt abgeführt werden. Die vom Harnstoff-Reaktor benötigte Wärmemenge muss dann zusätzlich bereitgestellt werden und die Energiebilanz des Prozesses wird ungünstiger. Des Weiteren wird bei den im Stand der Technik beschriebenen Verfahren eine erhebliche zusätzliche Wassermenge mit dem Carbamatstrom in die Harnstoff-Synthese eingetragen, wodurch das Gleichgewicht der Harnstoff-Bildungsreaktion ungünstig beeinflusst wird. Ohne zusätzlichen Wassereintrag liegt die Harnstoffausbeute im Idealfall bei ca. 45%. Jedes weitere eingetragene Wassermolekül verschlechtert die Ausbeute. Umfangreiche Information findet man in der Literatur, z.B. in S. Kawasumi, Equilibrium of the CO2-NH3-H2O-Urea System under High Temperature and Pressure. III. Effect of Water Added on Vapor-Liquid Equilibrium, Bull. Chem. Soc. Jap, Vol 26 (1953), No. 5, pp. 218-222.

US 1,670,341 betrifft ein Verfahren zur Herstellung von Harnstoff aus CO₂ und synthetischem NH₃.

US 3,310,376 offenbart ein Verfahren, bei dem CO₂ aus Synthesegas mit NH₃ bei einem Druck ausgewaschen wird, welcher auch bei der Synthese von NH₃ vorherrscht. Das Kopfprodukt aus dem Wäscher wird gereinigt und als Frischgas für die NH₃ Synthese eingesetzt.

US 3,674,847 betrifft eine integrierte NH₃- und Harnstoffsynthese. Das CO₂ aus dem rohen Synthesegas wird durch eine Anordnung aus Stripper - Harnstoffreaktor - Carbamat-Kondensor ausgewaschen. Die Reinigung des Synthesegases erfolgt durch Wäsche und unter Einsatz von Molekularsieb vor Abgabe an die NH₃ Synthese.

US 4,291,006 offenbart ein Reaktorkonzept für die Entfernung von CO₂ aus Synthesegasen mit Hilfe von wässrigem Ammoniak unter Bildung von Ammoniumcarbamat. Der Aufbau ist zweistufig mit Bodensektion und Filmabsorbersektion.

US 4,690,812 betrifft ein Verfahren zur Synthese von NH₃ und Harnstoff, bei dem keine direkte Integration verwirklicht wird. Es erfolgt eine Regeneration der Waschlösung in der CO₂-Wäsche nach Austrieb der Hauptmenge an CO₂ in der ersten Druckabsenkung mit der Prozessluft für den Sekundärreformer. Bei der kombinierten Herstellung von NH₃ und Harnstoff durch Umsetzung von kohlenwasserstoffhaltigen Gasen mit Wasserdampf in einem Steamreformer mit nachgeschaltetem Sekundärreformer wird das im gebildeten Gasgemisch vorhandene CO einer Konvertierung zu CO₂ unterzogen, worauf das nunmehr im Wesentlichen aus H₂, N₂ und CO₂ bestehende NH₃-Synthesegas einer Druckwäsche zur Entfernung saurer Verunreinigungen, insbesondere CO₂, mit einem physikalisch wirkenden Lösungsmittel unterworfen wird, woraufhin der Druck über dem beladenen Lösungsmittel zur Ausgasung von koabsorbierten Inerten auf einen ersten Zwischendruck und anschließend zur Entfernung der Hauptmenge des absorbierten CO₂, das zur Harnstoffsynthese herangezogen wird, auf einen zweiten Zwischendruck und schließlich zur Entfernung des Rest-CO₂ auf den Regenerierenddruck gesenkt wird, wonach das Lösungsmittel zur Druckwäsche zurückgeführt wird.

US 4,869,887 betrifft die Integration der Produktion von NH₃ und Harnstoff mit Hilfe adiabatischcr Rcformicrung einer Kohlenwasserstoffquelle. Dabei erfolgt eine Auswaschung von CO₂ aus dem rohen Synthesegas mit konzentrierter NH₃ Lösung unter Bildung von Ammoniumcarbamat.

US 6,231,827 offenbart ein Prozesskonzept mit Integration von NH₃- und Harnstoffsynthese. Die Entfernung von NH₃ aus dem Kreislaufgas erfolgt dabei unter CO₂ Auswaschung aus dem rohen Synthesegas in einem zweistufigen Fallfilmabsorber.

US 6,723,876 offenbart ein Prozesskonzept mit Integration von NH₃- und Harnstoffsynthese. Die Entfernung von NH₃ aus dem Kreislaufgas erfolgt mittels Wasserwäsche. Die Auswaschung von CO₂ aus dem rohen Synthesegas erfolgt mit konzentrierter NH₃-Lösung unter Bildung von Ammoniumcarbamat.

US 7,642,377 und US 8,481,786 betreffen die Kopplung von NH₃- und Hamstoffsysnthese über eine CO₂-Wäsche mit NH₃ bzw- wässrigem NH₃ und Zersetzung des im Harnstoffreaktor gebildeten Ammoniumcarbamats mittels Restwärme aus dem eintretenden Synthesegas. Das Strippen erfolgt thermisch mit Synthesegas, jedoch indirekt. Die Absorption von CO₂ erfolgt bei hohen Temperaturen und hohem Druck.

DE 32 39 605 A1 offenbart ein Verfahren zur kombinierten Herstellung von NH₃ und Harnstoff, bei dem das im Wesentlichen aus H₂, N₂ und CO₂ bestehende NH₃-Synthesegas einer Druckwäsche bei Temperaturen von höchstens Umgebungstemperatur zur Entfernung saurer Verunreinigung, insbesondere CO₂, mit einem physikalisch wirkenden Lösungsmittel unterworfen wird, woraufhin das beladene Lösungsmittel zur Ausgasung von Inerten teilentspannt und anschließend drucklos regeneriert und zur Druckwäsche zurückgeführt wird und das bei der Regenerierung freiwerdende CO₂ zur Harnstoffsynthese herangezogen wird. Dieses Verfahren ermöglicht jedoch nur eine geringfügige Reduzierung der Kompressionsarbeit. Zusätzlich wird in diesem Verfahren nur ein Teil des Kohlendioxids bei einem höheren Druck aus dem Lösungsmittel freigesetzt. Ein CO₂-Kompressor ist daher auch bei diesem Verfahren weiterhin erforderlich.

Die bekannten vollintegrierten Verfahren und Vorrichtungen zur Herstellung von Harnstoff sind nicht in jedem Punkt zufriedenstellend und es besteht ein Bedarf an verbesserten Verfahren und Vorrichtungen. Bisher ist keine produzierende vollintegrierte Anlage mit einer nennenswerten Leistung von beispielsweise 1000 Tagestonnen bekannt.

Diese Aufgabe wird durch den Gegenstand der Patentansprüche gelöst und ist in der Beschreibung offenbart.

Die Erfindung ist in den Ansprüchen definiert und betrifft ein Verfahren zur Herstellung von Harnstoff. Das erste Teil des Verfahren umfasst die folgende Schritte:
(a) Bereitstellen eines Rohsynthesegasstroms umfassend H₂, N₂ und CO₂;
(b) ggf. Verdichten des Rohsynthesegasstroms;
(c) Waschen des Rohsynthesegasstroms mit flüssigem NH₃ in einer ersten Waschkolonne (1a) unter Bildung eines an CO₂ angereicherten ersten Kondensats und eines an CO₂ abgereicherten vorgereinigten Synthesegasstroms;
(d) Abkühlen des vorgereinigten Synthesegasstroms und Abtrennen einer ersten flüssigen Phase;
(e) Waschen des vorgereinigten Synthesegasstroms mit flüssigem NH₃ in einer zweiten Waschkolonne (1b) unter Bildung eines an CO₂ angereicherten zweiten Kondensats und eines an CO₂ abgereicherten gereinigten Synthesegasstroms;
(f) Abkühlen des gereinigten Synthesegasstroms und Abtrennen einer zweiten flüssigen Phase;
(g) Waschen des gereinigten Synthesegasstroms mit flüssigem NH₃ in einer dritten Waschkolonne (1c) unter Bildung eines an CO₂ angereicherten dritten Kondensats und eines an CO₂ abgereicherten nachgereinigten Synthesegasstroms;
(h) Abkühlen des nachgereinigten Synthesegasstroms und Abtrennen einer dritten flüssigen Phase;
(i) Vereinen der ersten Kondensats aus Schritt (c), des zweiten Kondensats aus Schritt (e) und dritten Kondensats aus Schritt (g) unter Bildung eines Eduktstroms umfassend NH₃ und CO₂, welche ggf. gebunden in Form von Carbamat vorliegen können; und
(j) Synthetisieren von Harnstoff aus dem Eduktstrom unter Bildung einer wässrigen Harnstoffzusammensetzung und eines Restgasstroms umfassend nicht umgesetztes NH₃ und CO₂.

Das erfindungsgemäße Verfahren vermindert durch die spezielle Prozessführung die vorstehend beschriebenen Nachteile der integrierten Anlagenkonzepte signifikant und schafft eine Basis für die wirtschaftliche Umsetzung des Grundkonzeptes einer prozessseitig vollintegrierten NH₃/Harnstoff-Anlage.

Der Hauptvorteil des erfindungsgemäßen Konzeptes liegt in dem hier nur minimalen zusätzlichen Wassereintrag. So erfolgt das Waschen des Synthesegases in Schritten (c), (e) und (g) mit flüssigem NH₃, bevorzugt jedoch nicht mit wässriger NH₃-Lösung. Als Konsequenz wird im Vergleich mit dem konventionellen Referenzprozess nur eine geringfügig verringerte Harnstoffausbeute registriert. Da das erfindungsgemäße Konzept aber insgesamt mit geringeren CO₂-Verlusten verbunden ist, wird im Endeffekt mehr Harnstoff synthetisiert.

Ferner kann bei dem erfindungsgemäßen Verfahren die während der Abtrennung des CO₂ aus Rohsynthesegas mit NH₃ anfallende Reaktionsenthalpie anteilig wieder in den Prozess integriert werden. Ein anderer Teil der Reaktionsenthalpie könnte an anderen Stellen der integrierten NH₃- und Harnstoff-Synthese Verwendung finden, z.B. in der Vorwärmung des Kesselwassers. Die restliche für den Harnstoff-Prozess benötigte Wärme kann durch Modifikation des Dampfsystems des Gesamtkomplexes zur Verfügung gestellt werden. Eine nennenswerte Verbesserung des spezifischen Energieverbrauchs ist allerdings nach dem derzeitigen Stand der Untersuchungen nicht zu erwarten. Der spezifische Energieverbrauch des erfindungsgemäßen integrierten Verfahrens entspricht dadurch weitgehend dem herkömmlicher Verfahren.

Das erfindungsgemäße Verfahren führt durch den Entfall der konventionellen CO₂ Wäsche der NH₃-Anlage sowie des ansonsten für die Harnstoffsynthese zusätzlich erforderlichen CO₂-Verdichters samt seiner gesamten Peripherie zu einer gravierenden Reduzierung der Kapitalkosten eines Düngemittelkomplexes und ist damit wirtschaftlich sehr attraktiv.

Bei dem erfindungsgemäßen Verfahren wird schrittweise CO₂ aus dem Synthesegas unter Einsatz von flüssigem NH₃ entfernt (auskondensiert) und das nach Phasentrennung zurückbleibende Synthesegas auf diese Weise schnittweise an CO₂ abgcrcichcrt, d.h. gereinigt. Das unter Einsatz des flüssigen NH₃ gebildete Kondensat hingegen wird an CO₂ aus dem Synthesegas angereichert.

Bei dem erfindungsgemäßen Verfahren wird in Schritt (a) ein Synthesegas bereitgestellt, welches erfindungsgemäß als "*Rohsynthesegas*" bezeichnet wird. Dieses Rohsynthesegas unterscheidet sich hinsichtlich seiner Zusammensetzung, insbesondere Hinsichtlich seines Gehalts an CO₂, von dem gemäß Schritt (c) erhaltenen "*vorgereinigten Synthesegas*"*,* von dem gemäß Schritt (e) erhaltenen "*gereinigten Synthesegas*" sowie von dem gemäß Schritt (g) erhaltenen "*nachgereinigten Synthesegas*"*.* Grundsätzlich nimmt im Verlauf des erfindungsgemäßen Verfahrens der Gehalt an CO₂ im Synthesegas kontinuierlich immer weiter ab, bis der Gehalt an CO₂ schließlich so gering ist, dass das Synthesegas als "*Reinstsynthesegas*" für die Ammoniaksynthese eingesetzt werden kann.

In Schritt (a) des erfindungsgemäßen Verfahrens erfolgt das Bereitstellen eines Rohsynthesegasstroms umfassend H₂, N₂ und CO₂.

Dabei sind H₂, N₂ und CO₂ bevorzugt die Hauptbestandteile des Rohsynthesegasstroms. Gegebenenfalls kann der Rohsynthesegasstrom weitere, bevorzugt inerte Komponenten wie CH₄, Ar, CO und/oder He umfassen. Der Rohsynthesegasstrom wird bevorzugt aus Kohlenwasserstoffen, vorzugsweise aus Erdgas, Wasser in Form von Dampf. Geeignete Verfahren zur Erzeugung eines solchen Rohsynthesegasstroms sind einem Fachmann bekannt und es kann diesbezüglich beispielsweise vollumfänglich verwiesen werden auf A. Nielsen, I. Dybkjaer, Ammonia - Catalysis and Manufacture, Springer Berlin 1995, Kapitel 6, Seiten 202-326; M. Appl, Ammonia. Principles and Industrial Practice, WILEY-VCH Verlag GmbH 1999. In einer bevorzugten Ausführungsform wird zumindest ein Teil des Rohsynthesegasstroms durch Dampfreformierung und/oder durch autotherme Reformierung bereitgestellt.

Der in Schritt (a) des erfindungsgemäßen Verfahrens bereitgestellte Synthesegasstrom kann bereits herkömmlichen Aufbereitungsmaßnahmen für Synthesegas unterzogen worden sein, wie z.B. Heliumentfernung, Erdgasentschwefelung und/oder Konvertierung von CO zu CO₂.

Bevorzugt wurde der in Schritt (a) bereitgestellte Rohsynthesegasstrom zuvor bereits zumindest einer solchen Konvertierung von CO zu CO₂ unterzogen.

Besonders bevorzugt wurde der Rohsynthesegasstrom jedoch zuvor keinen weiteren Aufbereitungsmaßnahmen unterzogen, welche bei der herkömmlichen Bereitstellung von Synthesegas üblicherweise auf eine solche Konvertierung von CO zu CO₂ folgen, wie z.B. CO₂-Entfernung oder Endreinigung. Für eine Endreinigung kommen in herkömmlichen Prozessen insbesondere Methanisierung, Methanolisierung, kryogene Reinigung (Stickstoffwäsche), Kupferwäsche oder deren Kombinationen in Betracht. Solchen weiteren Aufbereitungsmaßnahmen wurde der in Schritt (a) bereitgestellte Rohsynthesegasstrom bevorzugt jedoch nicht unterzogen.

Der Gehalt an CO₂ im Rohsynthesegasstrom liegt bevorzugt im Bereich von 1 mol.-% bis 25 mol.-%, bevorzugter von 10 mol.-% bis 20 mol.-%, bezogen auf die gesamte Stoffmenge aller Bestandteile des Rohsynthesegasstroms.

Im Schritt (b) des erfindungsgemäßen Verfahrens erfolgt ggf. das Verdichten des Rohsynthesegasstroms. Geeignete Vorrichtungen zum Verdichten von Synthesegas sind einem Fachmann bekannt. Bevorzugt erfolgt erfindungsgemäß in Schritt (b) das Verdichten auf einen Druck, der oberhalb des üblichen Druckniveaus der Harnstoffsynthese liegt und hoch genug ist, um die Druckverluste im weiteren Verlauf des erfindungsgemäßen Verfahrens bis hin zur eigentlichen Harnstoffsynthese in Schritt (j) zu kompensieren.

Das übliche Druckniveau der Harnstoffsynthese in Schritt (j) liegt bei mindestens ca. 145 bar, so dass bevorzugt in Schritt (b) das Verdichten des Rohsynthesegasstroms auf einen Druck von mindestens 150 bar erfolgt, bevorzugt von mindestens 160 bar, bevorzugter von mindestens 170 bar, besonders bevorzugt jedoch nicht mehr als 190 bar.

Bei dem erfindungsgemäßen Verfahren wird der Synthesegasstrom schrittweise an CO₂ abgereichert. Dieses CO₂ wird in Form von Carbamat für die Harnstoffsynthese in Schritt (j) eingesetzt. Diese Abreicherung von CO₂ aus dem Synthesegas erfolgt erfindungsgemäß durch Auskondcnsicrcn mit flüssigem NH₃, wobei sich unter den Bedingungen des erfindungsgemäßen Verfahrens im so erhaltenen Kondensat zumindest anteilig Ammoniumcarbamat bildet. Das dabei eingesetzte NH₃, welches als Bestandteil des Ammoniumcarbamats vorliegt, ist global gesehen das andere der beiden Edukte für die Harnstoffsynthese in Schritt (j).

Bevorzugt erfolgen nach Schritt (b) des erfindungsgemäßen Verfahrens alle Maßnahmen, bei denen Zusammensetzungen prozessiert werden, welche mit CO₂ aus dem Synthesegas angereichert wurden, bei einem Druckniveau von mindestens 145 bar oder bei einem Druck, welcher leicht oberhalb des Synthesedrucks des Harnstoffs liegt und ausreichend hoch ist, um die Druckverluste zu überwinden.

Das auf diese Weise schrittweise an CO₂ abgereicherte Synthesegas umfasst schließlich im Wesentlichen nur noch H₂ und N₂ sowie ggf. Inertgase und wird dann erfindungsgemäß bevorzugt schließlich einem Ammoniakreaktor zugeführt, in dem die Synthese von NH₃ erfolgt. Das so synthetisierte NH₃ wird dann bevorzugt im verflüssigten Zustand zur schrittweisen Ausschleusung von CO₂ aus dem Synthesegas eingesetzt, wodurch eine Integration von Synthesegasreinigung, Ammoniaksynthese und Harnstoffsynthese möglich wird.

In Schritt (c) des erfindungsgemäßen Verfahrens erfolgt das Waschen des Rohsynthesegasstroms mit flüssigem NH₃ in einer ersten Waschkolonne (1a) unter Bildung eines an CO₂ angereicherten ersten Kondensats und eines an CO₂ abgereicherten vorgereinigten Synthesegasstroms. Bevorzugt wird dabei die Menge an flüssigem NH₃ so bemessen, dass unter den gegebenen Bedingungen keine übermäßig starke Erwärmung und Verdampfung der Flüssigkeit stattfindet. Das an CO₂ angereicherte erste Kondensat liegt flüssig vor und kann in der ersten Waschkolonne (1a) von dem gasförmigen an CO₂ abgereicherten vorgereinigten Synthesegasstrom abgetrennt werden.

Geeignete Waschkolonnen zum Waschen von Synthesegas mit flüssigem NH₃ sind einem Fachmann bekannt.

In Schritt (d) des erfindungsgemäßen Verfahrens erfolgt das Abkühlen des vorgcrcinigtcn Synthesegasstroms, vorzugsweise unter Einsatz eines Wärmetauschers (13), und das Abtrennen einer ersten flüssigen Phase, vorzugsweise unter Einsatz eines Abscheiders (22).

Bevorzugt wird die flüssige Phase aus Schritt (d) in die erste Waschkolonne (1a) in Schritt (c) rückgeführt, so dass Waschen des Rohsynthesegasstroms in Schritt (c) mit flüssigem NH₃ und zusätzlich mit der flüssigen Phase aus Schritt (d) erfolgt.

Geeignete Wärmetauscher zum Abkühlen von Synthesegas sind einem Fachmann bekannt.

Geeignete Abscheider zum Abtrennen einer flüssigen Phase von Synthesegas sind einem Fachmann bekannt.

Der Gehalt an CO₂ im vorgereinigten Synthesegasstrom aus Schritt (c) ist erfindungsgemäß geringer als im Rohsynthesegasstrom. Bevorzugt liegt der Gehalt an CO₂ im vorgereinigten Synthesegasstrom aus Schritt (c) im Bereich von 0,5 mol.-% bis 20,0 mol.-%, im Bereich von 0,5 mol.-% bis 10,0 mol.-%, im Bereich von 0,5 mol.-% bis 8,0 mol.-% oder im Bereich von 0,5 mol.-% bis 6,0 mol.-%, bezogen auf die gesamte Stoffmenge aller Bestandteile des vorgereinigten Synthesegasstroms.

Das an CO₂ angereicherte erste Kondensat aus Schritt (c) enthält NH₃ und CO₂ bevorzugt im Bereich von 5 mol.-% bis 90 mol.-%, bevorzugter im Bereich von 15 mol.-% bis 90 mol.-%, bevorzugter im Bereich von 25 mol.-% bis 90 mol.-%, bevorzugter im Bereich von 35 mol.-% bis 90 mol.-%, und noch bevorzugter im Bereich von 45 mol.-% bis 90 mol.-% , bezogen auf die gemeinsame Stoffmenge von NH₃ und CO₂, und bezogen auf die gesamte Stoffmenge aller Bestandteile des ersten Kondensats. Dabei liegen NH₃ und CO₂ zumindest anteilig gebunden in Form von Ammoniumcarbamat vor.

In Schritt (e) des erfindungsgemäßen Verfahrens erfolgt das Waschen des vorgereinigten Synthesegasstroms mit flüssigem NH₃ in einer zweiten Waschkolonne (1b) unter Bildung eines an CO₂ angereicherten zweiten Kondensats und eines an CO₂ abgereicherten gereinigten Synthesegasstroms. Das an CO₂ angereicherte zweite Kondensat liegt flüssig vor und kann in der zweiten Waschkolonne (1b) von dem gasförmigen an CO₂ abgereicherten gereinigten Synthesegasstrom abgetrennt werden.

In Schritt (f) des erfindungsgemäßen Verfahrens erfolgt das Abkühlen des gereinigten Synthesegasstroms, vorzugsweise unter Einsatz eines Wärmetauschers (14), und das Abtrennen einer zweiten flüssigen Phase, vorzugsweise unter Einsatz eines Abscheiders (23).

Der Gehalt an CO₂ im gereinigten Synthesegasstrom aus Schritt (e) ist erfindungsgemäß geringer als im vorgereinigten Synthesegasstrom. Bevorzugt liegt der Gehalt an CO₂ im gereinigten Synthesegasstrom aus Schritt (e) im Bereich von im Bereich von 0,5 mol.-% bis 15 mol.-%, im Bereich von 0,5 mol.-% bis 8,0 mol.-%, im Bereich von 0,5 mol.-% bis 6,0 mol.-% oder im Bereich von 0,5 mol.-% bis 4,0 mol.-%, bezogen auf die gesamte Stoffmenge aller Bestandteile des gereinigten Synthesegasstroms.

Das an CO₂ angereicherte zweite Kondensat aus Schritt (e) enthält NH₃ und CO₂ bevorzugt im Bereich von 5 mol.-% bis 90 mol.-%, bevorzugter im Bereich von 15 mol.-% bis 90 mol.-%, bevorzugter im Bereich von 25 mol.-% bis 90 mol.-%, bevorzugter im Bereich von 35 mol.-% bis 90 mol.-%, und noch bevorzugter im Bereich von 45 mol.-% bis 90 mol.-% , bezogen auf die gemeinsame Stoffmenge von NH₃ und CO₂, und bezogen auf die gesamte Stoffmenge aller Bestandteile des zweiten Kondensats. Dabei liegen NH₃ und CO₂ zumindest anteilig gebunden in Form von Ammoniumcarbamat vor.

In einer bevorzugten Ausführungsform wird die zweite flüssige Phase aus Schritt (f) in die zweite Waschkolonne (1b) in Schritt (e) rückgeführt, so dass das Waschen des vorgereinigten Synthesegasstroms in Schritt (e) mit flüssigem NH₃ und zusätzlich mit der zweiten flüssigen Phase erfolgt.

In Schritt (g) des erfindungsgemäßen Verfahrens erfolgt das Waschen des gereinigten Synthesegasstroms mit flüssigem NH₃ in einer dritten Waschkolonne (1c) unter Bildung eines an CO₂ angereicherten dritten Kondensats und eines an CO₂ abgereicherten nachgereinigten Synthesegasstroms. Das an CO₂ angereicherte dritte Kondensat liegt flüssig vor und kann in der dritten Waschkolonne (1c) von dem gasförmigen an CO₂ abgereicherten nachgereinigten Synthesegasstrom abgetrennt werden.

In Schritt (h) des erfindungsgemäßen Verfahrens erfolgt das Abkühlen des nachgereinigten Synthesegasstroms, vorzugsweise unter Einsatz eines Wärmetauschers (15), und das Abtrennen einer dritten flüssigen Phase, vorzugsweise unter Einsatz eines Abscheiders (24).

Der Gehalt an CO₂ im nachgereinigten Synthesegasstrom aus Schritt (g) ist erfindungsgemäß geringer als im gereinigten Synthesegasstrom. Bevorzugt liegt der Gehalt an CO₂ im nachgereinigten Synthesegasstrom aus Schritt (g) im Bereich von 0,5 mol.-% bis 1,0 mol.-%, bevorzugter im Bereich von 0,4 mol.-% bis 0,8 mol.-%, bevorzugter im Bereich von 0,3 mol.-% bis 0.6 mol.-%, bevorzugter im Bereich von 0,2 mol.-% bis 0,4 mol.-%, bevorzugter im Bereich von 0,1 mol.-% bis 0.2 mol.-% oder bevorzugter unterhalb von 0,5 mol.-% (500 ppm) oder der Gehalt an CO₂ im nachgereinigten Synthesegasstrom beträgt bevorzugter weniger als 0,1 mol.-%, bezogen auf die gesamte Stoffmenge aller Bestandteile des nachgereinigten Synthesegasstroms.

Das an CO₂ angereicherte dritte Kondensat aus Schritt (g) enthält NH₃ und CO₂ bevorzugt im Bereich von 5 mol.-% bis 90 mol.-%, bevorzugter im Bereich von 15 mol.-% bis 90 mol.-%, bevorzugter im Bereich von 25 mol.-% bis 90 mol.-%, bevorzugter im Bereich von 35 mol.-% bis 90 mol.-%,und noch bevorzugter im Bereich von 45 mol.-% bis 90 mol.-% , bezogen auf die gemeinsame Stoffmenge von NH₃ und CO₂, und bezogen auf die gesamte Stoffmenge aller Bestandteile des dritten Kondensats. Dabei liegen NH₃ und CO₂ zumindest anteilig gebunden in Form von Ammoniumcarbamat vor.

In einer bevorzugten Ausführungsform wird die dritte flüssige Phase aus Schritt (h) in die dritte Waschkolonne (1c) in Schritt (g) rückgeführt, so dass das Waschen des gereinigten Synthesegasstroms in Schritt (g) mit flüssigem NH₃ und zusätzlich mit der dritten flüssigen Phase erfolgt.

In Schritt (i) des erfindungsgemäßen Verfahrens erfolgt das Vereinen der ersten Kondensats aus Schritt (c), des zweiten Kondensats aus Schritt (e) und dritten Kondensats aus Schritt (g) unter Bildung eines Eduktstroms umfassend brutto NH₃ und CO₂. Dabei liegen NH₃ und CO₂ zumindest anteilig gebunden in Form von Ammoniumcarbamat vor.

In Schritt (j) des erfindungsgemäßen Verfahrens erfolgt dann schließlich das Synthetisieren von Harnstoff, vorzugsweise in einem Harnstoffreaktor (2), aus dem Eduktstrom unter Bildung einer wässrigen Harnstoffzusammensetzung. Da die Harnstoff-synthese üblicherweise nicht quantitativ verläuft, verbleibt zusätzlich ein Restgasstrom umfassend nicht umgesetztes NH₃ und CO₂. Ein Anteil von nicht umgesetzten Edukten befindes sich auch in der oben genannten wässrigen Harnstoffzusammensetzung.

Bevorzugt wird der Eduktstrom vor der Harnstoffsynthese unter Einsatz eines Wärmetauschers (16) erwärmt.

Sofern Druckverluste aufgetreten sind, kann der flüssige Eduktsrom zunächst verdichtet werden, bevorzugt mit einer Pumpe, bevorzugt mit einem Injektor, bevorzugt auf einen Druck von mindestens 100 bar, bevorzugter auf einen Druck von mindestens 120 bar, mindestens 140 bar oder mindestens 150 bar, aber leicht oberhalb des Druckes im Harnstoffreaktor (2).

Bevorzugt erfolgt die Synthese von Harnstoff bei einem Druck im Bereich von 100 bis 300 bar, bevorzugter im Bereich von 120 bis 200 bar oder im Bereich von 140 bis 160 bar. Die in Schritt (j) hergestellte wässrige Harnstoffzusammensetzung umfasst im Wesentlichen Harnstoff, Wasser, Ammoniumcarbamat, andere Ammoniumsalze wie Ammoniumhydrogencarbonat und Ammoniumcarbonat sowie ggf. nicht reagiertes NH₃ und CO₂. Das Ammoniumcarbamat soll thermisch zersetzt werden und das NH₃ sowie das CO₂ müssen aus der Lösung entfernt werden und können danach erneut für das Reaktionsverfahren zur Harnstoffsynthese bereitgestellt werden. Das Entfernen wird bevorzugt durch Strippen mit CO₂, mit NH₃, mit gereinigtem NH₃-Synthesegas oder mit dem Rohsynthesegas erreicht. Diese Verfahren sind Stand der Technik bei Harnstoffanlagen und dem Fachmann bekannt. Die Synthese von Harnstoff erfolgt bevorzugt in einer herkömmlichen Harnstoffanlage, deren Aufbau einem Fachmann bekannt ist.

In einer bevorzugten Ausführungsform umfasst das Synthetisieren des Harnstoffs in Schritt (j) die beiden Teilreaktionen:
- Bildung von Ammoniumcarbamat aus noch freiliegendem oder aus freiwerdendem NH₃ und CO₂; und
- Umsetzen des Ammoniumcarbamats zu Harnstoff und Wasser;
wobei die Energie für die endotherme zweite Teilreaktion zumindest teilweise aus der exothermen ersten Teilreaktion gedeckt wird. Da der Eduktstrom größtenteils aus Ammoniumcarbamat besteht, kann die exotherme Reaktion zur Bildung von Ammoniumcarbamat nicht vollständig den Energiebedarf für die endotherme Reaktion abdecken und deswegen wird der Harnstoffreaktor (2) bevorzugt zusätzlich von außen beheizt.

Ammoniumcarbamat wird überwiegend in der(den) Waschkolonne(n) gebildet. Im Reaktor entsteht allenfalls noch ein kleiner Bruchteil.

In einer bevorzugten Ausführungsform sind die in Schritt (c) eingesetzte erste Waschkolonne (1a) und die in Schritt (e) eingesetzte zweite Waschkolonne (1b) übereinander in einem gemeinsamen Gehäuse einer Waschkolonne (1) angeordnet, von welcher die Waschkolonne (1a) eine untere Sektion und die zweite Waschkolonne (1b) eine obere Sektion bildet.

In einer anderen bevorzugten Ausführungsform sind die in Schritt (c) eingesetzte erste Waschkolonne (1a) und die in Schritt (g) eingesetzte dritte Waschkolonne (1c) übereinander in einem gemeinsamen Gehäuse einer Waschkolonne (1) angeordnet, von welcher die Waschkolonne (1a) eine untere Sektion und die dritte Waschkolonne (1c) eine obere Sektion bildet.

In einer weiteren bevorzugten Ausführungsform sind die in Schritt (e) eingesetzte zweite Waschkolonne (1b) und die in Schritt (g) eingesetzte dritte Waschkolonne (1c) übereinander in einem gemeinsamen Gehäuse einer Waschkolonne (1) angeordnet, von welcher die zweite Waschkolonne (1b) eine untere Sektion und die dritte Waschkolonne (1c) eine obere Sektion bildet.

In einer besonders bevorzugten Ausführungsform sind die in Schritt (c) eingesetzte erste Waschkolonne (1a), die in Schritt (e) eingesetzte zweite Waschkolonne (1b) und die in Schritt (g) eingesetzte dritte Waschkolonne (1c) übereinander in einem gemeinsamen Gehäuse einer Waschkolonne (1) angeordnet, von welcher die Waschkolonne (1a) eine untere Sektion, die zweite Waschkolonne (1b) eine mittlere Sektion und die dritte Waschkolonne (1c) eine obere Sektion bildet.

In einer anderen bevorzugten Ausführungsform sind die erste Waschkolonne (1a), die zweite Waschkolonne (1b) und die dritte Waschkolonne (1c) jeweils für sich in voneinander getrennten Gehäusen angeordnet.

In einer bevorzugten Ausführungsform sind die Waschkolonnen (1a), (1b) und/oder (1c) in einem gemeinsamen Gehäuse angeordnet und zumindest eine der Waschkolonnen enthält
- zumindest eine Öffnung konfektioniert für den Zufluss einer flüssigen Phase,
- zumindest eine Öffnung konfektioniert für den Abfluss einer flüssigen Phase,
- zumindest eine Öffnung konfektioniert für das Zuströmen einer gasförmigen Phase, und/oder
- zumindest eine Öffnung konfektioniert für das Abströmen einer gasförmigen Phase.

Weiterhin umfasst das erfindungsgemäße Verfahren
- das Aufteilen des Rohsynthesegasstroms aus Schritt (b) in einen ersten Rohsynthesegasteilstrom und einen zweiten Rohsynthesegasteilstrom;
- das Strippen der wässrigen Harnstoffzusammensetzung aus Schritt (j) mit dem ersten Rohsynthesegasteilstrom unter Bildung eines Strippgasstroms;
- das Vereinen des Strippgasstroms mit dem zweiten Rohsynthesegasteilstrom; und
- das Abkühlen des so vereinten Rohsynthesegasstroms, vorzugsweise unter Einsatz eines Wärmetauschers (12), und das Abtrennen einer vierten flüssigen Phase, vorzugsweise unter Einsatz eines Abscheiders (21).

Bevorzugt wird dabei die gestrippte wässrige Harnstoffzusammensetzung zu einer Niederdruck-Aufbereitungsanlage (6) überführt und darin eine an CO₂ und NH₃ angereicherten fünfte flüssige Phase abgetrennt. Diese fünfte flüssige Phase wird bevorzugt in die zweite Waschkolonne (1b) in Schritt (e) zurückgeführt, so dass das Waschen des vorgereinigten Synthesegasstroms in Schritt (e) mit flüssigem NH₃, ggf. mit einer zweiten flüssigen Phase, und zusätzlich mit der fünften flüssigen Phase erfolgt.

Bevorzugt wird dabei die vierte flüssige Phase in die erste Waschkolonne (1a) in Schritt (c) zurückgeführt, so dass das Waschen des Rohsynthesegasstroms in Schritt (c) mit flüssigem NH₃, ggf. mit der ersten flüssigen Phase und zusätzlich mit der vierten flüssigen Phase erfolgt.

In einer bevorzugten Ausführungsform umfasst das erfindungsgemäße Verfahren das Rückführen der ersten flüssigen Phase aus Schritt (d) in die erste Waschkolonne (1a) in Schritt (c), so dass das Waschen des Rohsynthesegasstroms in Schritt (c) mit flüssigem NH₃ und zusätzlich mit der ersten flüssigen Phase erfolgt.

In einer bevorzugten Ausführungsform umfasst das erfindungsgemäße Verfahren das Vereinen des vorgereinigten Synthesegasstroms aus Schritt (c) mit dem Restgasstrom aus Schritt (j), so dass diese anschließend gemeinsam Schritt (d) zugeführt werden. Dazu wird der Restgasstrom bevorzugt zunächst abgekühlt, vorzugsweise unter Einsatz eines Wärmetauschers (17), und erst anschließend mit dem vorgereinigten Synthesegasstrom aus Schritt (c) vereint, vorzugsweise im Abscheider (22).

In einer bevorzugten Ausführungsform umfasst das erfindungsgemäße Verfahren das Abtrennen einer Waschlösung umfassend NH₃ von dem nachgereinigten Synthesegasstrom aus Schritt (h), vorzugsweise unter Einsatz einer vierten Waschkolonne (4), unter Bildung eines Reinstsynthesegasstroms umfassend nahezu ausschließlich H₂ und N₂.

Dabei wird zumindest ein Teil des CO₂ des nachgereinigten Synthesegasstroms aus Schritt (h) mit einem Lösungsmittel unter Bildung einer mit CO₂ angereicherten Waschlösung abgetrennt. Ziel dieses Schritts ist die Abtrennung des NH₃. Das CO₂ wird dabei auch abgetrennt, wobei der Anteil an CO₂ bevorzugt unterhalb von 500 ppm liegen soll. Der Gehalt an NH₃ im Gas soll bevorzugt unterhalb von 5.0 mol.-% liegen, bevorzugter unterhalb von 2 mol.-%. Ansonsten werden keine weiteren besonderen Anforderungen gestellt. Der Synthesegasstrom kann dabei gekühlt werden, vorzugsweise auf Temperaturen im Bereich von ∼0°C bis 20°C, z.B. ∼5°C.

In einer bevorzugten Ausführungsform umfasst das Lösungsmittel dabei H₂O. Dieses wird bevorzugt so lange im Kreis zirkuliert (anteilige Rückführung), bis die vorgegebenen Reinheiten des Reinstsynthesegases erreicht werden.

Der Gehalt an CO₂ im Reinstsynthesegasstrom ist geringer als im nachgereinigten Synthesegasstrom und liegt bevorzugt unterhalb von 500 ppm.

In einer bevorzugten Ausführungsform wird dabei die Waschlösung abgekoppelt aus (4) in die zweite Waschkolonne (1b) in Schritt (e) rückgeführt, so dass das Waschen des vorgereinigten Synthesegasstroms in Schritt (e) mit flüssigem NH₃, ggf. mit der zweiten flüssigen Phase, ggf. mit der fünften flüssigen Phase und zusätzlich mit der Waschlösung erfolgt.

Bevorzugt wird der Reinstsynthesegasstrom umfassend H₂ und N₂ über weitere Prozessstufen anschließend zu einem Ammoniakreaktor überführt und darin NH₃ synthetisiert.

Das NH₃ wird dabei aus zumindest einem Teil des H₂ und aus zumindest einem Teil des N₂ synthetisiert, welche in dem Reinstsynthesegasstrom enthalten sind. Dazu wird der Reinstsynthesegasstrom in einen Ammoniakreaktor geleitet.

Gegebenenfalls umfasst der Reinstsynthesegasstrom noch Reste von CO₂ und/oder CO, welche bevorzugt vor der Synthese von NH₃ beispielsweise durch kryogene Methoden oder durch eine Druckwechsel-Adsorption von dem Reinstsynthesegasstrom abgetrennt werden können. Bevorzugt werden diese Reste durch Methanisierung zu Methan umgesetzt. Geeignete Verfahren zur Hydrierung von CO und CO₂ zu Methan sind einem Fachmann bekannt. Durch die Methanisierung wird der Gehalt an CO, und dabei auch ggf. CO₂, in dem Reinstsynthesegasstrom verringert und der Gehalt an Methan in dem Reinstsynthesegasstrom erhöht.

Gegebenenfalls wird vor der Ammoniaksynthese der Reinstsynthesegasstrom auf einen erhöhten Druck verdichtet, vorzugsweise auf einen Druck im Bereich von 120 bis 250 bar, bevorzugter im Bereich von 180 bis 220 bar. Vorzugsweise weist der Ammoniakreaktor zumindest ein Katalysatorbett auf, welches von dem Reinstsynthesegasstrom nicht rein axial, sondern vorwiegend radial durchströmt wird, vorzugsweise von außen nach innen.

Bevorzugt wird danach zumindest ein Teil des im Ammoniakreaktor gebildeten NH₃ von dem resultierenden Produktstrom durch Abkühlen abgetrennt, wozu der Gasstrom bevorzugt zunächst einen Wärmetauscher und anschließend eine Kondensationsvorrichtung durchläuft. Dabei wird der Gasstrom abgekühlt, bevorzugt auf Temperaturen im Bereich von +5 °C bis -40 °C, bevorzugter im Bereich von 0 °C bis -35 °C, im Bereich von -5 °C bis -33 °C oder im Bereich von -10 °C bis -30 °C, so dass NH₃ unter den gegebenen Bedingungen auskondensiert und so von der Gasphase durch Phasentrennung abgetrennt werden kann.

Besonders bevorzugt wird das erfindungsgemäße Verfahren so betrieben, dass eine Wärmeübertragung zwischen Wärmetauschern (12) und (16), zwischen Wärmetauschern (13) und (16) sowie zwischen Wärmetauschern (17) und (16) erfolgt.

Bei dem erfindungsgemäßen Verfahren entfällt bevorzugt eine konventionelle CO₂ Wäsche der NH₃-Anlage sowie ein ansonsten für die Harnstoffsynthese zusätzlich erforderlicher CO₂-Verdichter samt seiner gesamten Peripherie.

Ein weiterer Aspekt der Erfindung betrifft eine Vorrichtung zur Herstellung von Harnstoff, wobei die Vorrichtung in den Ansprüchen definiert ist. Die folgenden miteinander in Wirkverbindung stehenden Komponenten werden beschrieben:
(A) eine Vorrichtung konfektioniert zum Bereitstellen eines Rohsynthesegasstroms umfassend H₂, N₂ und CO₂;
(B) ggf. einen Verdichter konfektioniert zum Verdichten des Rohsynthesegasstroms;
(C) eine erste Waschkolonne (1a) konfektioniert zum Waschen des Rohsynthesegasstroms mit flüssigem NH₃ unter Bildung eines an CO₂ angereicherten ersten Kondensats und eines an CO₂ abgereicherten vorgereinigten Synthesegasstroms;
(D) einen Wärmetauscher (13) konfektioniert zum Abkühlen des vorgereinigten Synthesegasstroms und einen Abscheider (22) konfektioniert zum Abtrennen einer ersten flüssigen Phase;
(E) eine zweite Waschkolonne (1b) konfektioniert zum Waschen des vorgereinigten Synthesegasstroms mit flüssigem NH₃ unter Bildung eines an CO₂ angereicherten zweiten Kondensats und eines an CO₂ abgereicherten gereinigten Synthesegasstroms;
(F) einen Wärmetauscher (14) konfektioniert zum Abkühlen des gereinigten Synthesegasstroms und einen Abscheider (23) konfektioniert zum Abtrennen einer zweiten flüssigen Phase;
(G) eine dritte Waschkolonne (1c) konfektioniert zum Waschen des gereinigten Synthesegasstroms mit flüssigem NH₃ unter Bildung eines an CO₂ angereicherten dritten Kondensats und eines an CO₂ abgereicherten nachgereinigten Synthesegasstroms;
(H) einen Wärmetauscher (15) konfektioniert zum Abkühlen des nachgereinigten Synthesegasstroms und einen Abscheider (24) konfektioniert zum Abtrennen einer dritten flüssigen Phase;
(I) Mittel zum Vereinen des ersten Kondensats aus (C), des zweiten Kondensats aus (E) und des dritten Kondensats aus (G) unter Bildung eines Eduktstroms; und
(J) einen Harnstoffreaktor (2) konfektioniert zum Synthetisieren von Harnstoff aus dem Eduktstrom unter Bildung einer wässrigen Harnstoffzusammensetzung und eines Restgasstroms umfassend nicht umgesetztes NH₃ und CO₂.

Alle im Zusammenhang mit dem erfindungsgemäßen Verfahren beschriebenen bevorzugten Ausführungsformen gelten analog für die erfindungsgemäße Vorrichtung und werden daher an dieser Stelle nicht wiederholt.

Die erfindungsgemäße Vorrichtung umfasst eine Vorrichtung konfektioniert zum Bereitstellen eines Rohsynthesegasstroms. Bevorzugt umfasst diese einen Dampfreformer und/oder einen autothermen Reformer. Umfasst die Vorrichtung einen autothermen Reformer, kann die Menge an CO₂, welche in dem autothermen Reformer gebildet wird, durch Variation von Reaktionsparametern wie beispielsweise dem Druck oder dem Dampf/Kohlenstoff-Verhältnis kontrolliert werden. Bevorzugt wird im autothermen Reformer gerade so viel CO₂ gebildet, dass das hergestellte NH₃ bevorzugt vollständig mit CO₂ zu Harnstoff umgesetzt werden kann.

Auch umfasst die erfindungsgemäße Vorrichtung
- Mittel konfektioniert zum Aufteilen des Rohsynthesegasstroms aus dem Verdichter (5) in einen ersten Rohsynthesegasteilstrom und einen zweiten Rohsynthesegasteilstrom;
- Mittel konfektioniert zum Strippen der wässrigen Harnstoffzusammensetzung aus dem Harnstoffreaktor (2) mit dem ersten Rohsynthesegasteilstrom unter Bildung eines Strippgasstroms;
- Mittel konfektioniert zum Vereinen des Strippgasstroms mit dem zweiten Rohsynthesegasteilstrom; und
- einen Wärmetauscher (12) konfektioniert zum Abkühlen des so vereinten Rohsynthesegasstroms und einen Abscheider (21) konfektioniert zum Abtrennen einer vierten flüssigen Phase.

Bevorzugt umfasst die erfindungsgemäße Vorrichtung Mittel konfektioniert zum Überführen der gestrippten wässrigen Harnstoffzusammensetzung zu einer Niederdruck-Aufbereitungsanlage (6) und eine solche Niederdruck-Aufbereitungsanlage (6) konfektioniert zum Abtrennen einer an CO₂ und NH₃ angereicherten fünften flüssigen Phase. Bevorzugt umfasst die Vorrichtung dabei zusätzlich Mittel konfektioniert zum Rückführen der fünften flüssigen Phase in die zweite Waschkolonne (1b), so dass das Waschen des vorgereinigten Synthesegasstroms in der zweiten Waschkolonne (1b) mit flüssigem NH₃ und zusätzlich mit der fünften flüssigen Phase erfolgen kann.

Bevorzugt umfasst die erfindungsgemäße Vorrichtung Mittel konfektioniert zum Rückführen der vierten flüssigen Phase in die erste Waschkolonne (1a), so dass das Waschen des Rohsynthesegasstroms in der ersten Waschkolonne (1a) mit flüssigem NH₃ und zusätzlich mit der vierten flüssigen Phase erfolgen kann.

Bevorzugt umfasst die erfindungsgemäße Vorrichtung Mittel konfektioniert zum Rückführen der ersten flüssigen Phase in die erste Waschkolonne (1a), so dass das Waschen des Rohsynthesegasstroms in der ersten Waschkolonne (1a) mit flüssigem NH₃ und zusätzlich mit der ersten flüssigen Phase erfolgen kann.

Bevorzugt umfasst die erfindungsgemäße Vorrichtung Mittel konfektioniert zum Vereinen des vorgereinigten Synthesegasstroms aus der ersten Waschkolonne (1a) mit dem Restgasstrom aus dem Harnstoffreaktor (2). Bevorzugt umfasst die erfindungsgemäße Vorrichtung dazu Mittel konfektioniert zum Abkühlen des Restgasstrom aus dem Harnstoffreaktor (2), vorzugsweise einen Wärmetauscher (17). Als Mittel konfektioniert zum Vereinen des vorgereinigten Synthesegasstroms aus der ersten Waschkolonne (1a) mit dem Restgasstrom aus dem Harnstoffreaktor (2) dient vorzugsweise Abscheider (22).

Bevorzugt umfasst die erfindungsgemäße Vorrichtung eine vierte Waschkolonne (4) konfektioniert zum Abtrennen einer Waschlösung umfassend NH₃ von dem nachgereinigten Synthesegasstrom aus der dritten Waschkolonne (1c) unter Bildung eines Reinstsynthesegasstroms umfassend H₂ und N₂.

Bevorzugt umfasst die erfindungsgemäße Vorrichtung Mittel konfektioniert zum Rückführen der Waschlösung aus (4) in die zweite Waschkolonne (1b), so dass das Waschen des vorgereinigten Synthesegasstroms in der zweiten Waschkolonne (1b) mit flüssigem NH₃ und zusätzlich mit der Waschlösung aus (4) erfolgt.

Bevorzugt umfasst die erfindungsgemäße Vorrichtung einen Ammoniakreaktor konfektioniert zum Synthetisieren von NH₃ sowie Mittel konfektioniert zum Überführen des Reinstsynthesegasstroms umfassend H₂ und N₂ in den Ammoniakreaktor.

Bevorzugt umfasst die erfindungsgemäße Vorrichtung Mittel konfektioniert zum Verdichten, vorzugsweise eine Pumpe oder einen Injektor (7), und Mittel konfektioniert zum Erwärmen des Eduktstroms, vorzugsweise einen Wärmetauscher (16).

Bevorzugt umfasst die erfindungsgemäße Vorrichtung Mittel konfektioniert zum Rückführen der zweiten flüssigen Phase in die zweite Waschkolonne (1b), so dass das Waschen des vorgereinigten Synthesegasstroms in der zweiten Waschkolonne (1b) mit flüssigem NH₃ und zusätzlich mit der zweiten flüssigen Phase erfolgen kann.

Bevorzugt umfasst die erfindungsgemäße Vorrichtung Mittel konfektioniert zum Rückführen der dritten flüssigen Phase in die dritte Waschkolonne (1c), so dass das Waschen des gereinigten Synthesegasstroms in der dritten Waschkolonne (1c) mit flüssigem NH₃ und zusätzlich mit der dritten flüssigen Phase erfolgen kann.

Besonders bevorzugt ist die erfindungsgemäße Vorrichtung so konfektioniert, dass eine Wärmeübertragung zwischen Wärmetauschern (12) und (16), zwischen Wärmetauschern (13) und (16) sowie zwischen Wärmetauschern (17) und (16) erfolgen kann.

Die erfindungsgemäße Vorrichtung eignet sich besonders zum Durchführen des erfindungsgemäßen Verfahrens. Ein weiterer Aspekt der Erfindung betrifft daher die Verwendung der erfindungsgemäßen Vorrichtung in dem erfindungsgemäßen Verfahren.

Bevorzugt umfasst die erfindungsgemäße Vorrichtung keine Anlage zur konventionellen CO₂ Wäsche, wie sie in konventionellen Anlagen zur Synthese von NH₃ häufig nach der CO Konvertierung eingesetzt werden, sowie keinen ansonsten für die Harnstoffsynthese zusätzlich erforderlichen CO₂-Verdichter samt seiner gesamten Peripherie. Bevorzugt umfasst die erfindungsgemäße Vorrichtung keinen Verdichter konfektioniert zur Verdichtung von reinem CO₂ (CO₂-Kompressor).

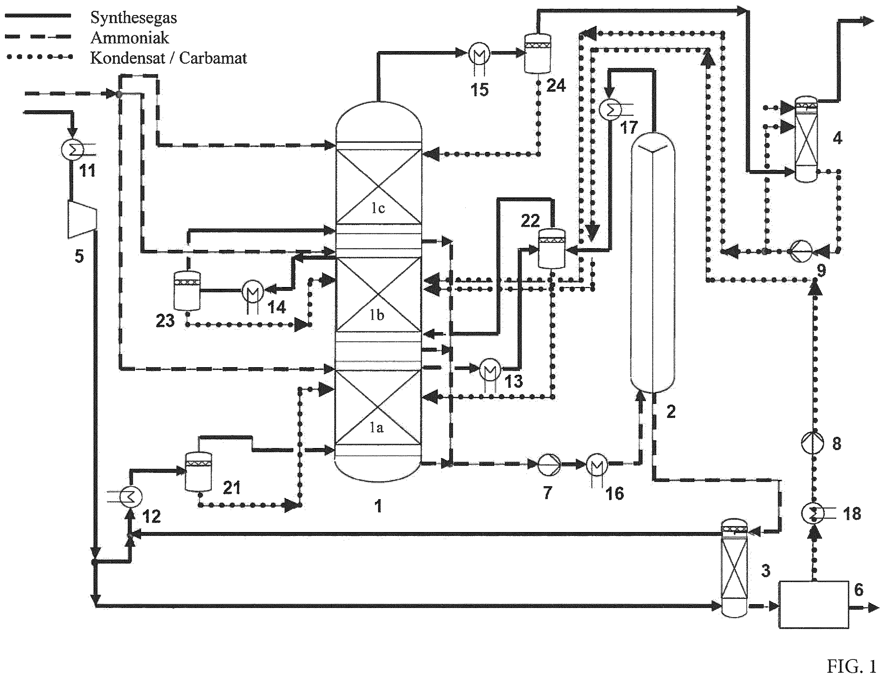

Die Erfindung wird nachfolgend anhand des Verfahrensschemas gemäß Abbildung 1 erläutert.

Das Rohsynthesegas nach der CO-Konvertierung wird im Wärmetauscher (11) abgekühlt und im Synthesegas-Verdichter (5) auf einen Druck komprimiert, der leicht oberhalb des Niveaus der Harnstoffsynthese liegt und hoch genug ist, um die Druckverluste bis zur eigentlichen Harnstoffsynthese zu kompensieren. Anschließend wird das Rohsynthesegas zunächst geteilt und etwa zur Hälfte für das Strippen der aus dem Harnstoffreaktor (2) kommenden Harnstofflösung im Hochdruck-Stripper (3) verwendet. Die Strippgase und das restliche Rohsynthesegas werden wieder vereinigt, im Wärmetauscher (12) abgekühlt und im Abscheider (21) in gasförmige und flüssige Phasen getrennt. Die Gasphasc wird in die untere Sektion (1a) der Waschkolonne (1) geleitet, wo sie mit flüssigem NH₃ und dem Kondensat aus dem Abscheider (21) und aus dem Abscheider (22) in Kontakt gebracht wird. Die Menge an flüssigem NH₃ ist so bemessen, dass dabei keine übermäßig starke Erwärmung und Verdampfung der flüssigen Phase stattfindet.

Die endgültige Reinheit des CO₂ wird in der oberen Sektion der Waschkolonne (1) erreicht. Das Synthesegas aus der unteren Sektion (1a) wird im Wärmetauscher (13) gekühlt und in den Abscheider (22) geleitet. Dort wird das Synthesegas mit der Gasphase aus dem Harnstoffreaktor (2) über Wärmetauscher (17) vereinigt. Nach der Phasentrennung wird das Kondensat aus dem Abscheider (22) in die untere Sektion (1a) der Waschkolonne (1) geleitet und die Gasphase wird in der mittleren Sektion der Waschkolonne (1) wieder mit flüssigem NH₃ im Kontakt gebracht. Zusätzlich zu dem flüssigen NH₃ wird die mittlere Sektion (1b) der Waschkolonne (1) mit Kondensat aus dem Abscheider (23), Kondensat aus der Niederdruck-Aufbereitung (6) des Harnstoffes sowie mit der Waschlösung aus der Nachwaschkolonne (4) gespeist.

Nach der Abkühlung in Wärmetauscher (14) und Phasentrennung in Abscheider (23) wird das Synthesegas in die obere Sektion (1c) der Waschkolonne (1) geführt, wo es durch Waschen mit flüssigem NH₃ vom restlichen CO₂ befreit wird. Nach Abkühlung im Wärmetauscher (15) und Phasentrennung im Abscheider (24) wird das CO₂-abgereinigte Synthesegas der Nachwaschkolonne (4) zugeführt, in der das überschüssige NH₃ zurückgehalten und über die Pumpe (9) als Ammoniaklösung rezykliert wird. Die Kondensatphasen aus allen drei Sektionen des der Waschkolonne (1) werden kombiniert, die Druckverluste über die Pumpe (7) ausgeglichen und das Gemisch im Wärmetauscher (16) auf die Reaktionstemperatur erwärmt, bevor es in den Harnstoffreaktor (2) eintritt. Die nicht umgesetzten, gasförmigen Edukte (hauptsächlich NH₃ und CO₂) verlassen den Harnstoffreaktor (2) über den Kopf und werden rezykliert. Die noch NH₃- und CO₂-haltige Harnstofflösung aus dem Harnstoffreaktor (2) wird in den Hochdruck-Stripper (3) geleitet und mit Rohsynthesegas gestrippt. Die resultierende Harnstofflösung wird in die Niederdruck-Aufbereitung (6) weitergeleitet. Der aus der Niederdruck-Aufbereitung (6) ausgeschleuste Strom an Kondensat / Carbamat wird bevorzugt über Wärmetauscher (18) und Pumpe (8) zur mittleren Sektion (1b) der Waschkolonne (1) rückgeführt.

Das vorgeschlagene Konzept bietet zahlreiche Möglichkeiten zur Wärmeintegration, anteilig innerhalb der Harnstoffsynthese und anteilig außerhalb. Zur prozessinternen Wärmeintegration zählt die Wärmeübertragung zwischen Wärmetauschern (12) und (16), zwischen Wärmetauschern (13) und (16) sowie zwischen Wärmetauschern (17) und (16); die restliche nicht prozessintern verwendbare Niedertemperaturwärme aus den Wärmetauschern (11) bis (15) und (17) kann außerhalb der Harnstoffsynthese beispielsweise für die Vorwärmung des Kesselspeisewassers verwendet werden.

Die für die Aufwärmung des Reaktionsgemisches für die eigentliche Harnstoff-synthese benötigte Wärmemenge sowie die für den Mehrbedarf des Synthesegaskompressors und ggf. für den Mehrbedarf der Kältemaschine benötigte Dampfmenge kann mit begrenzten Modifikationen des Dampfsystems in NH₃/Harnstoff-Komplexen verfügbar gemacht werden. Des Weiteren kann auch die von der CO₂-Wäsche in der konventionellen Prozessführung beanspruchte Wärme sowie der bisherige Dampfbedarf für den Antrieb des CO₂-Verdichters durch das neue Dampfsystem genutzt werden.

Mit den Annahmen, dass die Modifikationen des Dampfsystems im NH₃-Harnstoff-Komplex und der Ersatz des Carbamatkondensators in der Harnstoff-Anlage durch die NH₃-Waschkolonne weitgehend kostenneutral sind, ergibt sich durch den Wegfall des CO₂-Verdichters einschließlich seiner Zusatzeinrichtungen (Antriebsturbine, Zwischenkühler, Ölsystem, etc.) und durch den Wegfall der aMDEA®-basierten CO₂-Wäsche eine sehr substanzielle Reduzierung der Anlageninvestitionskosten.

### Bezugszeichenliste:

- 1: - Waschkolonne mit unterer, mittlerer und oberer Sektion
- 1a: - erste Waschkolonne, untere Sektion der Waschkolonne
- 1b: - zweite Waschkolonne, mittlere Sektion der Waschkolonne
- 1c: - dritte Waschkolonne, obere Sektion der Waschkolonne
- 2: - Harnstoffreaktor
- 3: - Hochdruck-Stripper
- 4: - vierte Waschkolonne, Nachwaschkolonne
- 5: - Synthcscgas-Vcrdichtcr
- 6: - Niederdruck-Aufbereitungsanlage
- 7: - Pumpe oder Injektor
- 8: - Pumpe oder Injektor
- 9: - Pumpe oder Injektor
- 11: - Wärmetauscher
- 12: - Wärmetauscher
- 13: - Wärmetauscher
- 14: - Wärmetauscher
- 15: - Wärmetauscher
- 16: - Wärmetauscher
- 17: - Wärmetauscher
- 18: - Wärmetauscher
- 21: - Abscheider
- 22: - Abscheider
- 23: - Abscheider
- 24: - Abscheider

## Patentansprüche

1. Ein Verfahren zur integrierten Herstellung von Ammoniak und Harnstoff umfassend die folgenden Schritte:
(a) Bereitstellen eines Rohsynthesegasstroms umfassend H₂, N₂ und CO₂;
(b) Verdichten des Rohsynthesegasstroms;
(c) Waschen des Rohsynthesegasstroms mit flüssigem NH₃ in einer ersten Waschkolonne (1a) unter Bildung eines an CO₂ angereicherten ersten Kondensats und eines an CO₂ abgereicherten vorgereinigten Synthesegasstroms;
(d) Abkühlen des vorgereinigten Synthesegasstroms und Abtrennen einer ersten flüssigen Phase;
(e) Waschen des vorgereinigten Synthesegasstroms mit flüssigem NH₃ in einer zweiten Waschkolonne (1b) unter Bildung eines an CO₂ angereicherten zweiten Kondensats und eines an CO₂ abgereicherten gereinigten Synthesegasstroms;
(f) Abkühlen des gereinigten Synthesegasstroms und Abtrennen einer zweiten flüssigen Phase;
(g) Waschen des gereinigten Synthesegasstroms mit flüssigem NH₃ in einer dritten Waschkolonne (1c) unter Bildung eines an CO₂ angereicherten dritten Kondensats und eines an CO₂ abgereicherten nachgereinigten Synthesegasstroms;
(h) Abkühlen des nachgereinigten Synthesegasstroms und Abtrennen einer dritten flüssigen Phase;
(i) Vereinen der ersten Kondensats aus Schritt (c), des zweiten Kondensats aus Schritt (e) und dritten Kondensats aus Schritt (g) unter Bildung eines Eduktstroms umfassend NH₃ und CO₂, welche zumindest anteilig gebunden in Form von Ammoniumcarbamat vorliegen können; und
(j) Synthetisieren von Harnstoff aus dem Eduktstrom unter Bildung einer wässrigen Harnstoffzusammensetzung und eines Restgasstroms umfassend nicht umgesetztes NH₃ und CO_{2;}
wobei das Verfahren ferner umfasst:
- das Aufteilen des Rohsynthesegasstroms aus Schritt (b) in einen ersten Rohsynthesegasteilstrom und einen zweiten Rohsynthesegasteilstrom;
- das Strippen der wässrigen Harnstoffzusammensetzung aus Schritt (j) mit dem ersten Rohsynthesegasteilstrom unter Bildung eines Strippgasstroms;
- das Vereinen des Strippgasstroms mit dem zweiten Rohsynthesegasteilstrom; und
- das Abkühlen des so vereinten Rohsynthesegasstroms und Abtrennen einer vierten flüssigen Phase.

2. Das Verfahren nach Anspruch 1, wobei die erste Waschkolonne (1a) und die zweite Waschkolonne (1b) übereinander in einem gemeinsamen Gehäuse einer Waschkolonne (1) angeordnet sind, von welcher die Waschkolonne (1a) eine untere Sektion und die zweite Waschkolonne (1b) eine obere Sektion bildet; und/oder wobei die erste Waschkolonne (1a) und die dritte Waschkolonne (1c) übereinander in einem gemeinsamen Gehäuse einer Waschkolonne (1) angeordnet sind, von welcher die erste Waschkolonne (1a) eine untere Sektion und die dritte Waschkolonne (1c) eine obere Sektion bildet; und/oder wobei die zweite Waschkolonne (1b) und die dritte Waschkolonne (1c) übereinander in einem gemeinsamen Gehäuse einer Waschkolonne (1) angeordnet sind, von welcher die zweite Waschkolonne (1b) eine untere Sektion und die dritte Waschkolonne (1c) eine obere Sektion bildet; und/oder wobei die erste Waschkolonne (1a), die zweite Waschkolonne (1b) und die dritte Waschkolonne (1c) übereinander in einem gemeinsamen Gehäuse einer Waschkolonne (1) angeordnet sind, von welcher die erste Waschkolonne (1a) eine untere erste Sektion, die zweite Waschkolonne (1b) eine mittlere zweite Sektion und die dritte Waschkolonne (1c) eine obere dritte Sektion bildet; und/oder wobei die erste Waschkolonne (1a), die zweite Waschkolonne (1b) und die dritte Waschkolonne (1c) übereinander in einem gemeinsamen Gehäuse einer Waschkolonne (1) angeordnet sind, von welcher die erste Waschkolonne (1a) eine untere Sektion, die zweite Waschkolonne (1b) eine mittlere Sektion und die dritte Waschkolonne (1c) eine obere Sektion bildet.

3. Das Verfahren nach einem der vorstehenden Ansprüche, wobei die erste Waschkolonne (1a), die zweite Waschkolonne (1b) und die dritte Waschkolonne (1c) jeweils für sich in voneinander getrennten Gehäusen angeordnet sind;
oder wobei das Waschen in Schritten (c), (e) und (g) nicht mit wässriger NH₃-Lösung ausgeführt wird;
oder wobei der Rohsynthesegasstrom in Schritt (b) auf einen Druck von mindestens 150 bar, oder auf mindestens 160 bar, oder auf mindestens 170 bar, oder auf mindestens 190 bar, oder auf mindestens 220 bar, oder auf mindestens 250 bar verdichtet wird;
oder wobei das Abkühlen des vorgereinigten Synthesegasstroms in Schritt (d) unter Einsatz eines Wärmetauschers (13) erfolgt; und/oder wobei das Abkühlen des gereinigten Synthesegasstroms in Schritt (f) unter Einsatz eines Wärmetauschers (14) erfolgt.

4. Das Verfahren nach einem der vorstehenden Ansprüche, wobei zumindest eine der Waschkolonnen (1a), (1b) und/oder (1c)
- zumindest eine Öffnung konfektioniert für den Zufluss einer flüssigen Phase,
- zumindest eine Öffnung konfektioniert für den Abfluss einer flüssigen Phase,
- zumindest eine Öffnung konfektioniert für das Zuströmen einer gasförmigen Phase, und/oder
- zumindest eine Öffnung konfektioniert für das Abströmen einer gasförmigen Phase enthält.

5. Das Verfahren nach einem der vorstehenden Ansprüche, wobei der Gehalt an CO₂ im vorgereinigten Synthesegasstrom aus Schritt (c) im Bereich von 0,5 mol.-% bis 20,0 mol.-%, im Bereich von 0,5 mol.-% bis 10,0 mol.-%, im Bereich von 0,5 mol.-% bis 8,0 mol.-% oder im Bereich von 0,5 mol.-% bis 6,0 mol.-% liegt, bezogen auf die gesamte Stoffmenge aller Bestandteile des vorgereinigten Synthesegasstroms; und/oder wobei der Gehalt an NH₃ und CO₂ im ersten Kondensat aus Schritt (c) im Bereich von 5 mol.-% bis 90 mol.-%, im Bereich von 15 mol.-% bis 90 mol.-%, im Bereich von 25 mol.-% bis 90 mol.-%, im Bereich von 35 mol.-% bis 90 mol.-%, oder im Bereich von 45 mol.-% bis 90 mol.-% liegt, bezogen auf die gemeinsame Stoffmenge von NH₃ und CO₂, und bezogen auf die gesamte Stoffmenge aller Bestandteile des ersten Kondensats, wobei NH₃ und CO₂ zumindest anteilig gebunden in Form von Ammoniumcarbamat vorliegen.

6. Das Verfahren nach einem der vorstehenden Ansprüche, wobei der Gehalt an CO₂ im gereinigten Synthesegasstrom aus Schritt (e) im Bereich von 0,5 mol.-% bis 15,0 mol.-%, im Bereich von 0,5 mol.-% bis 8,0 mol.-%, im Bereich von 0,5 mol.-% bis 6,0 mol.-% oder im Bereich von 0,5 mol.-% bis 4,0 mol.-% liegt, bezogen auf die gesamte Stoffmenge aller Bestandteile des gereinigten Synthesegasstroms; und/oder wobei der Gehalt an NH₃ und CO₂ im zweiten Kondensat aus Schritt (e) im Bereich von 5 mol.-% bis 90 mol.-%, im Bereich von 15 mol.-% bis 90 mol.-%, im Bereich von 25 mol.-% bis 90 mol.-%, im Bereich von 35 mol.-% bis 90 mol.-%, oder im Bereich von 45 mol.-% bis 90 mol.-% liegt, bezogen auf die gemeinsame Stoffmenge von NH₃ und CO₂, und bezogen auf die gesamte Stoffmenge aller Bestandteile des zweiten Kondensats, wobei NH₃ und CO₂ zumindest anteilig gebunden in Form von Ammoniumcarbamat vorliegen.

7. Das Verfahren nach einem der vorstehenden Ansprüche, wobei der Gehalt an CO₂ im nachgereinigten Synthesegasstrom aus Schritt (g) im Bereich von 0,5 mol.-% bis 1,0 mol.-%, im Bereich von 0,4 mol.-% bis 0,8 mol.-%, im Bereich von 0,3 mol.-% bis 0.6 mol.-%, im Bereich von 0,2 mol.-% bis 0,4 mol.-% oder im Bereich von 0,1 mol.-% bis 0.2 mol.-% liegt oder weniger als 0,1 mol.-% beträgt, bezogen auf die gesamte Stoffmenge aller Bestandteile des nachgereinigten Synthesegasstroms; und/oder wobei der Gehalt an NH₃ und CO₂ im dritten Kondensat aus Schritt (g) im Bereich von 5 mol.-% bis 90 mol.-%, im Bereich von 15 mol.-% bis 90 mol.-%, im Bereich von 25 mol.-% bis 90 mol.-%, im Bereich von 35 mol.-% bis 90 mol.-%, oder im Bereich von 45 mol.-% bis 90 mol.-% liegt, bezogen auf die gemeinsame Stoffmenge von NH₃ und CO₂, und bezogen auf die gesamte Stoffmenge aller Bestandteile des dritten Kondensats, wobei NH₃ und CO₂ zumindest anteilig gebunden in Form von Ammoniumcarbamat vorliegen.

8. Das Verfahren nach einem der vorhergehenden Ansprüche, wobei der vereinte Rohsynthesegasstrom unter Einsatz eines Wärmetauschers (12) abgekühlt wird.

9. Das Verfahren nach einem der Ansprüche 1 bis 7, umfassend das Überführen der gestrippten wässrigen Harnstoffzusammensetzung zu einer Niederdruck-Aufbereitungsanlage (6) und in dieser das Abtrennen einer an CO₂ und NH₃ angereicherten fünften flüssigen Phase, insbesondere ferner umfassend das Rückführen der fünften flüssigen Phase in die zweite Waschkolonne (1b) in Schritt (e), so dass das Waschen des vorgereinigten Synthesegasstroms in Schritt (e) mit flüssigem NH₃ und zusätzlich mit der fünften flüssigen Phase erfolgt.

10. Das Verfahren nach einem der vorhergehenden Ansprüche, umfassend das Rückführen der vierten flüssigen Phase in die erste Waschkolonne (1a) in Schritt (c), so dass das Waschen des Rohsynthesegasstroms in Schritt (c) mit flüssigem NH₃ und zusätzlich mit der vierten flüssigen Phase erfolgt.

11. Das Verfahren nach einem der vorstehenden Ansprüche, umfassend das Vereinen des vorgereinigten Synthesegasstroms aus Schritt (c) mit dem Restgasstrom aus Schritt (j); ferner umfassend wenigstens einen der folgenden Schritte:
wobei der Restgasstrom aus Schritt (j) vor der Vereinigung mit dem vorgereinigten Synthesegasstrom aus Schritt (c) unter Einsatz eines Wärmetauschers (17) abgekühlt wird;
oder umfassend das Abtrennen einer Waschlösung umfassend NH₃ von dem nachgereinigten Synthesegasstrom aus Schritt (h) unter Einsatz einer vierten Waschkolonne (4) unter Bildung eines Reinstsynthesegasstroms umfassend H₂ und N₂, wobei der Reinstsynthesegasstrom insbesondere einen Anteil von CO₂ unterhalb von 500 ppm und ein Gehalt an NH₃ von weniger als 5.0 mol-% enthält;
wobei das Verfahren ferner umfasst: Rückführen der Waschlösung in die zweite Waschkolonne (1b) in Schritt (e), so dass das Waschen des vorgereinigten Synthesegasstroms in Schritt (e) mit flüssigem NH₃ und zusätzlich mit der Waschlösung erfolgt; ferner optional auch umfassend das Überführen des Reinstsynthesegasstroms umfassend H₂ und N₂ zu einem Ammoniakreaktor und Synthetisieren von NH₃;
wobei das Verfahren optional ferner umfasst: Rückführen der ersten flüssigen Phase aus Schritt (d) in die erste Waschkolonne (1a) in Schritt (c), so dass das Waschen des Rohsynthesegasstroms in Schritt (c) mit flüssigem NH₃ und zusätzlich mit der ersten flüssigen Phase erfolgt; und/oder Rückführen der zweiten flüssigen Phase aus Schritt (f) in die zweite Waschkolonne (1b) in Schritt (e), so dass das Waschen des vorgereinigten Synthesegasstroms in Schritt (e) mit flüssigem NH₃ und zusätzlich mit der zweiten flüssigen Phase erfolgt; und/oder Rückführen der dritten flüssigen Phase aus Schritt (h) in die dritte Waschkolonne (1c) in Schritt (g), so dass das Waschen des gereinigten Synthesegasstroms in Schritt (g) mit flüssigem NH₃ und zusätzlich mit der dritten flüssigen Phase erfolgt;
oder Komprimieren mit Hilfe einer Pumpe und/oder das Erwärmen des Eduktstroms aus Schritt (i); und/oder wobei die Harnstoffsynthese in Schritt (j) bei einem Druck im Bereich von 100 bis 300 bar, insbesondere im Bereich von 120 bis 200 bar oder im Bereich von 140 bis 160 bar erfolgt.

12. Das Verfahren nach einem der vorstehenden Ansprüche, wobei der Eduktstrom aus Schritt (i) vor der Harnstoffsynthese in Schritt (j) unter Einsatz eines Wärmetauschers (16) erwärmt wird; und/oder wobei eine Wärmeübertragung zwischen Wärmetauschern (12) und (16), zwischen Wärmetauschern (13) und (16) sowie zwischen Wärmetauschern (17) und (16) erfolgt.

13. Eine Vorrichtung zur Herstellung von Harnstoff, wobei die Vorrichtung die folgenden miteinander in Wirkverbindung stehenden Komponenten umfasst:
(A) eine Vorrichtung konfektioniert zum Bereitstellen eines Rohsynthesegasstroms umfassend H₂, N₂ und CO₂;
(B) einen Verdichter konfektioniert zum Verdichten des Rohsynthesegasstroms;
(C) eine erste Waschkolonne (1a) konfektioniert zum Waschen des Rohsynthesegasstroms mit flüssigem NH₃ unter Bildung eines an CO₂ angereicherten ersten Kondensats und eines an CO₂ abgereicherten vorgereinigten Synthesegasstroms;
(D) einen Wärmetauscher (13) konfektioniert zum Abkühlen des vorgereinigten Synthesegasstroms und einen Abscheider (22) konfektioniert zum Abtrennen einer ersten flüssigen Phase;
(E) eine zweite Waschkolonne (1b) konfektioniert zum Waschen des vorgereinigten Synthesegasstroms mit flüssigem NH₃ unter Bildung eines an CO₂ angereicherten zweiten Kondensats und eines an CO₂ abgereicherten gereinigten Synthesegasstroms;
(F) einen Wärmetauscher (14) konfektioniert zum Abkühlen des gereinigten Synthesegasstroms und einen Abscheider (23) konfektioniert zum Abtrennen einer zweiten flüssigen Phase;
(G) eine dritte Waschkolonne (1c) konfektioniert zum Waschen des gereinigten Synthesegasstroms mit flüssigem NH₃ unter Bildung eines an CO₂ angereicherten dritten Kondensats und eines an CO₂ abgereicherten nachgereinigten Synthesegasstroms;
(H) einen Wärmetauscher (15) konfektioniert zum Abkühlen des nachgereinigten Synthesegasstroms und einen Abscheider (24) konfektioniert zum Abtrennen einer dritten flüssigen Phase;
(I) Mittel zum Vereinen der ersten Kondensats aus (C), des zweiten Kondensats aus (E) und dritten Kondensats aus (G) unter Bildung eines Eduktstroms; und
(J) einen Harnstoffreaktor (2) konfektioniert zum Synthetisieren von Harnstoff aus dem Eduktstrom unter Bildung einer wässrigen Harnstoffzusammensetzung und eines Restgasstroms umfassend nicht umgesetztes NH₃ und CO₂;
wobei die Vorrichtung eingerichtet ist zum Aufteilen des Rohsynthesegasstroms aus dem Verdichter (5) in einen ersten Rohsynthesegasteilstrom und einen zweiten Rohsynthesegasteilstrom;
und eingerichtet ist zum Strippen der gebildeten wässrigen Harnstoffzusammensetzung mit dem ersten Rohsynthesegasteilstrom unter Bildung eines Strippgasstroms;
und eingerichtet ist zum Vereinen des Strippgasstroms mit dem zweiten Rohsynthesegasteilstrom; und
eingerichtet ist zum Abkühlen des so vereinten Rohsynthesegasstroms mittels eines Wärmetauschers (12) und zum Abtrennen einer vierten flüssigen Phase mittels eines Abscheiders (21).

14. Die Vorrichtung nach dem vorhergehenden Anspruch, wobei die Vorrichtung konfektioniert zum Bereitstellen eines Rohsynthesegasstroms einen Dampfreformer und/oder einen autothermen Reformer umfasst; und/oder wobei die Vorrichtung keinen Verdichter konfektioniert zur Verdichtung von reinem CO₂ umfasst; und/oder wobei die Vorrichtung einen Ammoniakreaktor konfektioniert zur Synthese von Ammoniak umfasst.

15. Verwendung einer Vorrichtung nach einem der vorhergehenden Vorrichtungsansprüche in einem Verfahren nach einem der vorhergehenden Verfahrensansprüche.

## Claims

1. A process for integrated production of ammonia and urea comprising the steps of:
(a) providing a crude synthesis gas stream comprising H₂, N₂ and CO₂;
(b) compressing the crude synthesis gas stream;
(c) scrubbing the crude synthesis gas stream with liquid NH₃ in a first scrubbing column (1a) to form a CO₂-enriched first condensate and a CO₂-depleted prepurified synthesis gas stream;
(d) cooling the prepurified synthesis gas stream and separating a first liquid phase;
(e) scrubbing the prepurified synthesis gas stream with liquid NH₃ in a second scrubbing column (1b) to form a CO₂-enriched second condensate and a CO₂-depleted purified synthesis gas stream;
(f) cooling the purified synthesis gas stream and separating a second liquid phase;
(g) scrubbing the purified synthesis gas stream with liquid NH₃ in a third scrubbing column (1c) to form a CO₂-enriched third condensate and a CO₂-depleted postprepurified synthesis gas stream;
(h) cooling the postpurified synthesis gas stream and separating a third liquid phase;
(i) combining the first condensate from step (c), the second condensate from step (e) and the third condensate from step (g) to form a reactant stream comprising NH₃ and CO₂ which may be at least partly in a bound state in the form of ammonium carbamate; and
(j) synthesizing urea from the reactant stream to form an aqueous urea composition and a residual gas stream comprising unconverted NH₃ and CO₂;
wherein the process further comprises:
- dividing the crude synthesis gas stream from step (b) into a first crude synthesis gas substream and a second crude synthesis gas substream;
- stripping the aqueous urea composition from step (j) with the first crude synthesis gas substream to form a stripping gas stream;
- combining the stripping gas stream with the second crude synthesis gas substream; and
- cooling the thus-combined crude synthesis gas stream and separating a fourth liquid phase.

2. The process as claimed in claim 1, wherein the first scrubbing column (1a) and the second scrubbing column (1b) are arranged one above another in a common housing of a scrubbing column (1), of which the scrubbing column (1a) forms a lower section and the second scrubbing column (1b) forms an upper section; and/or wherein the first scrubbing column (1a) and the third scrubbing column (1c) are arranged one above another in a common housing of a scrubbing column (1), of which the first scrubbing column (1a) forms a lower section and the third scrubbing column (1c) forms an upper section; and/or wherein the second scrubbing column (1b) and the third scrubbing column (1c) are arranged one above another in a common housing of a scrubbing column (1), of which the second scrubbing column (1b) forms a lower section and the third scrubbing column (1c) forms an upper section; and/or wherein the first scrubbing column (1a), the second scrubbing column (1b) and the third scrubbing column (1c) are arranged one above another in a common housing of a scrubbing column (1), of which the first scrubbing column (1a) forms a lower first section, the second scrubbing column (1b) forms a middle second section and the third scrubbing column (1c) forms an upper third section; and/or wherein the first scrubbing column (1a), the second scrubbing column (1b) and the third scrubbing column (1c) are arranged one above another in a common housing of a scrubbing column (1), of which the first scrubbing column (1a) forms a lower section, the second scrubbing column (1b) forms a middle section and the third scrubbing column (1c) forms an upper section.

3. The process as claimed in any of the preceding claims, wherein the first scrubbing column (1a), the second scrubbing column (1b) and the third scrubbing column (1c) are each arranged independently in separate housings;
or wherein the scrubbing in steps (c), (e) and (g) is not performed with aqueous NH₃ solution;
or wherein the crude synthesis gas stream in step (b) is compressed to a pressure of at least 150 bar, or to at least 160 bar, or to at least 170 bar, or to at least 190 bar, or to at least 220 bar, or to at least 250 bar;
or wherein the cooling of the prepurified synthesis gas stream in step (d) is carried out using a heat exchanger (13); and/or wherein the cooling of the purified synthesis gas stream in step (f) is carried out using a heat exchanger (14).

4. The process as claimed in any of the preceding claims, wherein at least one of the scrubbing columns (1a), (1b) and/or (1c) contains
- at least one opening adapted for inflow of a liquid phase,
- at least one opening adapted for outflow of a liquid phase,
- at least one opening adapted for supplying a gaseous phase and/or
- at least one opening adapted for discharging a gaseous phase.

5. The process as claimed in any of the preceding claims, wherein the content of CO₂ in the prepurified synthesis gas stream from step (c) is in the range from 0.5 mol% to 20.0 mol%, in the range from 0.5 mol% to 10.0 mol%, in the range from 0.5 mol% to 8.0 mol% or in the range from 0.5 mol% to 6.0 mol% based on the total amount of substance of all constituents of the prepurified synthesis gas stream; and/or wherein the content of NH₃ and CO₂ in the first condensate from step (c) is in the range from 5 mol% to 90 mol%, in the range from 15 mol% to 90 mol%, in the range from 25 mol% to 90 mol%, in the range from 35 mol% to 90 mol%, or in the range from 45 mol% to 90 mol% based on the total amount of substance of NH₃ and CO₂ and based on the total amount of substance of all constituents of the first condensate, wherein NH₃ and CO₂ are at least partly in a bound state in the form of ammonium carbamate.

6. The process as claimed in any of the preceding claims, wherein the content of CO₂ in the purified synthesis gas stream from step (e) is in the range from 0.5 mol% to 15.0 mol%, in the range from 0.5 mol% to 8.0 mol%, in the range from 0.5 mol% to 6.0 mol% or in the range from 0.5 mol% to 4.0 mol% based on the total amount of substance of all constituents of the purified synthesis gas stream; and/or wherein the content of NH₃ and CO₂ in the second condensate from step (e) is in the range from 5 mol% to 90 mol%, in the range from 15 mol% to 90 mol%, in the range from 25 mol% to 90 mol%, in the range from 35 mol% to 90 mol%, or in the range from 45 mol% to 90 mol% based on the total amount of substance of NH₃ and CO₂ and based on the total amount of substance of all constituents of the second condensate, wherein NH₃ and CO₂ are at least partly in a bound state in the form of ammonium carbamate.

7. The process as claimed in any of the preceding claims, wherein the content of CO₂ in the postpurified synthesis gas stream from step (g) is in the range from 0.5 mol% to 1.0 mol%, in the range from 0.4 mol% to 0.8 mol%, in the range from 0.3 mol% to 0.6 mol%, in the range from 0.2 mol% to 0.4 mol% or in the range from 0.1 mol% to 0.2 mol% or is less than 0.1 mol% based on the total amount of substance of all constituents of the postpurified synthesis gas stream; and/or wherein the content of NH₃ and CO₂ in the third condensate from step (g) is in the range from 5 mol% to 90 mol%, in the range from 15 mol% to 90 mol%, in the range from 25 mol% to 90 mol%, in the range from 35 mol% to 90 mol%, or in the range from 45 mol% to 90 mol% based on the total amount of substance of NH₃ and CO₂ and based on the total amount of substance of all constituents of the third condensate, wherein NH₃ and CO₂ are at least partly in a bound state in the form of ammonium carbamate.

8. The process as claimed any of the preceding claims, wherein the combined crude synthesis gas stream is cooled using a heat exchanger (12).

9. The process as claimed in any of claims 1 to 7, comprising transferring the stripped aqueous urea composition into a low-pressure workup plant (6) and therein separating a CO₂- and NH₃-enriched fifth liquid phase, in particular further comprising recycling the fifth liquid phase into the second scrubbing column (1b) in step (e), so that the scrubbing of the prepurified synthesis gas stream in step (e) is effected with liquid NH₃ and additionally with the fifth liquid phase.

10. The process as claimed in any of the preceding claims, comprising recycling the fourth liquid phase into the first scrubbing column (1a) in step (c), so that the scrubbing of the crude synthesis gas stream in step (c) is effected with liquid NH₃ and additionally with the fourth liquid phase.

11. The process as claimed in any of the preceding claims, comprising combining the prepurified synthesis gas stream from step (c) with the residual gas stream from step (j);
or wherein prior to combining with the prepurified synthesis gas stream from step (c) the residual gas stream from step (j) is cooled using a heat exchanger (17);
or comprising separating a scrubbing solution comprising NH₃ from the postpurified synthesis gas stream from step (h) using a fourth scrubbing column (4) to form an ultrahigh purity synthesis gas stream comprising H₂ and N₂, wherein the ultrahigh purity synthesis gas stream in particular contains a proportion of CO₂ below 500 ppm and a content of NH₃ of less than 5.0 mol%;
or comprising recycling the scrubbing solution into the second scrubbing column (1b) in step (e), so that the scrubbing of the prepurified synthesis gas stream in step (e) is effected with liquid NH₃ and additionally with the scrubbing solution; and/or comprising transferring the ultrahigh purity synthesis gas stream comprising H₂ and N₂ into an ammonia reactor and synthesizing NH₃;
or comprising recycling the first liquid phase from step (d) into the first scrubbing column (1a) in step (c), so that the scrubbing of the crude synthesis gas stream in step (c) is effected with liquid NH₃ and additionally with the first liquid phase; and/or comprising recycling the second liquid phase from step (f) into the second scrubbing column (1b) in step (e), so that the scrubbing of the prepurified synthesis gas stream in step (e) is effected with liquid NH₃ and additionally with the second liquid phase; and/or comprising recycling the third liquid phase from step (h) into the third scrubbing column (1c) in step (g), so that the scrubbing of the purified synthesis gas stream in step (g) is effected with liquid NH₃ and additionally with the third liquid phase;
or comprising compressing using a pump and/or heating the reactant stream from step (i); and/or wherein the urea synthesis in step (j) is carried out at a pressure in the range from 100 to 300 bar, more preferably in the range from 120 to 200 bar or in the range from 140 to 160 bar.

12. The process as claimed in any of the preceding claims, wherein prior to the urea synthesis in step (j) the reactant stream from step (i) is heated using a heat exchanger (16); and/or wherein heat transfer between heat exchangers (12) and (16), between heat exchangers (13) and (16) and between heat exchangers (17) and (16) is effected.

13. An apparatus for producing urea, wherein the apparatus comprises the following operatively interconnected components:
(A) an apparatus adapted for providing a crude synthesis gas stream comprising H₂, N₂ and CO₂;
(B) a compressor adapted for compressing the crude synthesis gas stream;
(C) a first scrubbing column (1a) adapted for scrubbing the crude synthesis gas stream with liquid NH₃ to form a CO₂-enriched first condensate and a CO₂-depleted prepurified synthesis gas stream;
(D) a heat exchanger (13) adapted for cooling the prepurified synthesis gas stream and a separator (22) adapted for separating a first liquid phase;
(E) a second scrubbing column (1b) adapted for scrubbing the prepurified synthesis gas stream with liquid NH₃ to form a CO₂-enriched second condensate and a CO₂-depleted purified synthesis gas stream;
(F) a heat exchanger (14) adapted for cooling the purified synthesis gas stream and a separator (23) adapted for separating a second liquid phase;
(G) a third scrubbing column (1c) adapted for scrubbing the purified synthesis gas stream with liquid NH₃ to form a CO₂-enriched third condensate and a CO₂-depleted postpurified synthesis gas stream;
(H) a heat exchanger (15) adapted for cooling the postpurified synthesis gas stream and a separator (24) adapted for separating a third liquid phase;
(I) means for combining the first condensate from (C), the second condensate from (E) and the third condensate from (G) to form a reactant stream; and
(J) a urea reactor (2) adapted for synthesizing urea from the reactant stream to form an aqueous urea composition and a residual gas stream comprising unconverted NH₃ and CO₂;
wherein the apparatus is adapted for dividing the crude synthesis gas stream from step b) into a first crude synthesis gas sub stream and a second crude synthesis gas sub stream;
and is adapted for stripping the aqueous urea composition from step (j) with the first crude synthesis gas substream to form a stripping gas stream;
and is adapted for combining the stripping gas stream with the second crude synthesis gas substream; and
is adapted for cooling the thus-combined crude synthesis gas stream and separating a fourth liquid phase.

14. The apparatus as claimed in the preceding claim, wherein the apparatus adapted for providing a crude synthesis gas stream comprises a steam reformer and/or an autothermal reformer; and/or wherein the apparatus does not comprise a compressor adapted for compressing pure CO₂; and/or wherein the apparatus comprises an ammonia reactor adapted for synthesizing ammonia.

15. The use of an apparatus as claimed in any of the preceding apparatus claims in a process as claimed in any of the preceding process claims.

## Revendications

1. Procédé pour la préparation intégrée d'ammoniac et d'urée comprenant les étapes suivantes :
(a) mise à disposition d'un flux de gaz de synthèse brut comprenant H₂, N₂ et CO₂;
(b) compression du flux de gaz de synthèse brut ;
(c) lavage du flux de gaz de synthèse brute avec du NH₃ liquide dans une première colonne de lavage (1a) avec formation d'un premier condensat enrichi en CO₂et d'un flux de gaz de synthèse prépurifié appauvri en CO₂;
(d) refroidissement du flux de gaz de synthèse prépurifié et séparation d'une première phase liquide ;
(e) lavage du flux de gaz de synthèse prépurifié avec du NH₃ liquide dans une deuxième colonne de lavage (1b) avec formation d'un deuxième condensat enrichi en CO₂ et d'un flux de gaz de synthèse purifié appauvri en CO₂;
(f) refroidissement du flux de gaz de synthèse purifié et séparation d'une deuxième phase liquide ;
(g) lavage du flux de gaz de synthèse purifié avec du NH₃ liquide dans une troisième colonne de lavage (1c) avec formation d'un troisième condensat enrichi en CO₂ et d'un flux de gaz de synthèse post-purifié appauvri en CO₂;
(h) refroidissement du flux de gaz de synthèse post-purifié et séparation d'une troisième phase liquide ;
(i) réunion du premier condensat de l'étape (c), du deuxième condensat de l'étape (e) et du troisième condensat de l'étape (g) avec formation d'un flux d'éduit comprenant NH₃ et CO₂, lesquels peuvent être présents de manière au moins partiellement liée sous forme de carbamate d'ammonium ; et
(j) synthèse d'urée à partir du flux d'éduit avec formation d'une composition aqueuse d'urée et d'un flux de gaz résiduel comprenant NH₃ et CO₂ n'ayant pas réagi ;
le procédé comprenant en outre :
- la division du flux de gaz de synthèse brut de l'étape (b) en un premier flux partiel de gaz de synthèse brut et un deuxième flux partiel de gaz de synthèse brut ;
- le strippage de la composition aqueuse d'urée de l'étape (j) avec le premier flux partiel de gaz de synthèse brut avec formation d'un flux de gaz strippé ;
- la réunion du flux de gaz strippé avec le deuxième flux partiel de gaz de synthèse brut ; et
- le refroidissement du flux de gaz de synthèse brut ainsi réuni et la séparation d'une quatrième phase liquide.

2. Procédé selon la revendication 1, la première colonne de lavage (1a) et la deuxième colonne de lavage (1b) étant disposées l'une sur l'autre dans un boîtier commun d'une colonne de lavage (1), dont la colonne de lavage (1a) forme une section inférieure et la deuxième colonne de lavage (1b) forme une section supérieure ; et/ou la première colonne de lavage (1a) et la troisième colonne de lavage (1c) étant disposées l'une sur l'autre dans un boîtier commun d'une colonne de lavage (1), dont la première colonne de lavage (1a) forme une section inférieure et la troisième colonne de lavage (1c) forme une section supérieure ; et/ou la deuxième colonne de lavage (1b) et la troisième colonne de lavage (1c) étant disposées l'une sur l'autre dans un boîtier commun d'une colonne de lavage (1), dont la deuxième colonne de lavage (1b) forme une section inférieure et la troisième colonne de lavage (1c) forme une section supérieure ; et/ou la première colonne de lavage (1a), la deuxième colonne de lavage (1b) et la troisième colonne de lavage (1c) étant disposées l'une sur l'autre dans un boîtier commun d'une colonne de lavage (1), dont la première colonne de lavage (1a) forme une première section inférieure, la deuxième colonne de lavage (1b) forme une deuxième section médiane et la troisième colonne de lavage (1c) forme une troisième section supérieure ; et/ou la première colonne de lavage (1a), la deuxième colonne de lavage (1b) et la troisième colonne de lavage (1c) étant disposées l'une sur l'autre dans un boîtier commun d'une colonne de lavage (1), dont la première colonne de lavage (1a) forme une section inférieure, la deuxième colonne de lavage (1b) forme une section médiane et la troisième colonne de lavage (1c) forme une section supérieure.

3. Procédé selon l'une quelconque des revendications précédentes, la première colonne de lavage (1a), la deuxième colonne de lavage (1b) et la troisième colonne de lavage (1c) étant à chaque fois disposées individuellement dans des boîtiers séparés les uns des autres ;
ou le lavage dans les étapes (c), (e) et (g) n'étant pas mis en œuvre avec une solution aqueuse de NH₃ ;
ou le flux de gaz de synthèse brut dans l'étape (b) étant comprimé à une pression d'au moins 150 bars, ou d'au moins 160 bars, ou d'au moins 170 bars, ou d'au moins 190 bars, ou d'au moins 220 bars, ou d'au moins 250 bars ;
ou le refroidissement du flux de gaz de synthèse prépurifié dans l'étape (d) est réalisé avec l'utilisation d'un échangeur de chaleur (13) ; et/ou le refroidissement du flux de gaz de synthèse purifié dans l'étape (f) est réalisé avec l'utilisation d'un échangeur de chaleur (14).

4. Procédé selon l'une quelconque des revendications précédentes, au moins l'une des colonnes de lavage (1a), (1b) et/ou (1c) contenant
- au moins une ouverture conditionnée pour l'afflux d'une phase liquide,
- au moins une ouverture conditionnée pour l'évacuation d'une phase liquide,
- au moins une ouverture conditionnée pour l'apport d'une phase gazeuse, et/ou
- au moins une ouverture conditionnée pour l'échappement d'une phase gazeuse.

5. Procédé selon l'une quelconque des revendications précédentes, la teneur en CO₂ dans le flux de gaz de synthèse prépurifié de l'étape (c) se situant dans la plage de 0,5 % en moles à 20,0 % en moles, dans la plage de 0,5 % en moles à 10,0 % en moles, dans la plage de 0,5 % en moles à 8,0 % en moles ou dans la plage de 0,5 % en moles à 6,0 % en moles, par rapport à la quantité totale de matière de tous les ingrédients du flux de gaz de synthèse prépurifié ; et/ou la teneur en NH₃ et CO₂ dans le premier condensat de l'étape (c) se situant dans la plage de 5 % en moles à 90 % en moles, dans la plage de 15 % en moles à 90 % en moles, dans la plage de 25 % en moles à 90 % en moles, dans la plage de 35 % en moles à 90 % en moles ou dans la plage de 45 % en moles à 90 % en moles, par rapport à la quantité commune de matière de NH₃ et CO₂, et par rapport à la quantité totale de matière de tous les ingrédients du premier condensat, NH₃ et CO₂ étant présents de manière au moins partiellement liée sous forme de carbamate d'ammonium.

6. Procédé selon l'une quelconque des revendications précédentes, la teneur en CO₂ dans le flux de gaz de synthèse purifié de l'étape (e) se situant dans la plage de 0,5 % en moles à 15,0 % en moles, dans la plage de 0,5 % en moles à 8,0 % en moles, dans la plage de 0,5 % en moles à 6,0 % en moles ou dans la plage de 0,5 % en moles à 4,0 % en moles, par rapport à la quantité totale de matière de tous les ingrédients du flux de gaz de synthèse purifié ; et/ou la teneur en NH₃ et CO₂ dans le deuxième condensat de l'étape (e) se situant dans la plage de 5 % en moles à 90 % en moles, dans la plage de 15 % en moles à 90 % en moles, dans la plage de 25 % en moles à 90 % en moles, dans la plage de 35 % en moles à 90 % en moles ou dans la plage de 45 % en moles à 90 % en moles, par rapport à la quantité commune de matière de NH₃ et CO₂, et par rapport à la quantité totale de matière de tous les ingrédients du deuxième condensat, NH₃ et CO₂ étant présents de manière au moins partiellement liée sous forme de carbamate d'ammonium.

7. Procédé selon l'une quelconque des revendications précédentes, la teneur en CO₂ dans le flux de gaz de synthèse post-purifié de l'étape (g) se situant dans la plage de 0,5 % en moles à 1,0 % en moles, dans la plage de 0,4 % en moles à 0,8 % en moles, dans la plage de 0,3 % en moles à 0,6 % en moles, dans la plage de 0,2 % en moles à 0,4 % en moles ou dans la plage de 0,1 % en moles à 0,2 % en moles ou étant inférieure à 0,1 % en moles, par rapport à la quantité totale de matière de tous les ingrédients du flux de gaz de synthèse post-purifié ; et/ou la teneur en NH₃ et CO₂ dans le troisième condensat de l'étape (g) se situant dans la plage de 5 % en moles à 90 % en moles, dans la plage de 15 % en moles à 90 % en moles, dans la plage de 25 % en moles à 90 % en moles, dans la plage de 35 % en moles à 90 % en moles ou dans la plage de 45 % en moles à 90 % en moles, par rapport à la quantité commune de matière de NH₃ et CO₂, et par rapport à la quantité totale de matière de tous les ingrédients du troisième condensat, NH₃ et CO₂ étant présents de manière au moins partiellement liée sous forme de carbamate d'ammonium.

8. Procédé selon l'une quelconque des revendications précédentes, le flux de gaz de synthèse brut réuni étant refroidi avec l'utilisation d'un échangeur de chaleur (12).

9. Procédé selon l'une quelconque des revendications 1 à 7, comprenant le transfert de la composition aqueuse d'urée strippée à une installation de traitement (6) sous pression réduite et dans celle-ci, la séparation d'une cinquième phase liquide enrichie en CO₂ et NH₃, en particulier comprenant en outre le recyclage de la cinquième phase liquide dans la deuxième colonne de lavage (1b) dans l'étape (e), de sorte que le lavage du flux de gaz de synthèse prépurifié dans l'étape (e) est réalisé avec du NH₃ liquide et de plus avec la cinquième phase liquide.

10. Procédé selon l'une quelconque des revendications précédentes, comprenant le recyclage de la quatrième phase liquide dans la première colonne de lavage (1a) dans l'étape (c), de sorte que le lavage du flux de gaz de synthèse brut dans l'étape (c) est réalisé avec du NH₃ liquide et de plus avec la quatrième phase liquide.

11. Procédé selon l'une quelconque des revendications précédentes, comprenant la réunion du flux de gaz de synthèse prépurifié de l'étape (c) avec le flux de gaz résiduel de l'étape (j) ; comprenant en outre au moins l'une des étapes suivantes :
le flux de gaz résiduel de l'étape (j) étant refroidi avec l'utilisation d'un échangeur de chaleur (17) avant la réunion avec le flux de gaz de synthèse prépurifié de l'étape (c) ;
ou comprenant la séparation d'une solution de lavage comprenant du NH₃ d'avec le flux de gaz de synthèse post-purifié de l'étape (h) avec l'utilisation d'une quatrième colonne de lavage (4) avec formation d'un flux de gaz de synthèse extra pur comprenant H₂ et N₂, le flux de gaz de synthèse extra pur contenant en particulier une proportion de CO₂ inférieure à 500 ppm et une teneur en NH₃ inférieure à 5,0 % en moles ;
le procédé comprenant en outre : le recyclage de la solution de lavage dans la deuxième colonne de lavage (1b) dans l'étape (e), de sorte que le lavage du flux de gaz de synthèse prépurifié dans l'étape (e) est réalisé avec du NH₃ liquide et de plus avec la solution de lavage ; en outre éventuellement comprenant également le transfert du flux de gaz de synthèse extra pur comprenant H₂ et N₂ à un réacteur d'ammoniac et la synthèse de NH₃ ;
le procédé comprenant éventuellement en outre : le recyclage de la première phase liquide de l'étape (d) dans la première colonne de lavage (1a) dans l'étape (c), de sorte que le lavage du flux de gaz de synthèse brut dans l'étape (c) est réalisé avec du NH₃ liquide et de plus avec la première phase liquide ; et/ou le recyclage de la deuxième phase liquide de l'étape (f) dans la deuxième colonne de lavage (1b) dans l'étape (e), de sorte que le lavage du flux de gaz de synthèse prépurifié dans l'étape (e) est réalisé avec du NH₃ liquide et de plus avec la deuxième phase liquide ; et/ou le recyclage de la troisième phase liquide de l'étape (h) dans la troisième colonne de lavage (1c) dans l'étape (g), de sorte que le lavage du flux de gaz de synthèse purifié dans l'étape (g) est réalisé avec du NH₃ liquide et de plus avec la troisième phase liquide ;
ou la compression au moyen d'une pompe et/ou le chauffage du flux d'éduit de l'étape (i) ; et/ou la synthèse d'urée dans l'étape (j) est réalisée à une pression dans la plage de 100 à 300 bars, en particulier dans la plage de 120 à 200 bars ou dans la plage de 140 à 160 bars.

12. Procédé selon l'une quelconque des revendications précédentes, le flux d'éduit de l'étape (i) étant chauffé avant la synthèse d'urée dans l'étape (j) avec utilisation d'un échangeur de chaleur (16) ; et/ou un transfert de chaleur entre les échangeurs de chaleur (12) et (16), entre les échangeurs de chaleur (13) et (16) ainsi qu'entre les échangeurs de chaleur (17) et (16) est réalisé.

13. Dispositif pour la préparation d'urée, le dispositif comprenant les composants suivants en liaison active les uns avec les autres :
(A) un dispositif conditionné pour la mise à disposition d'un flux de gaz de synthèse brut comprenant H₂, N₂ et CO₂ ;
(B) un compresseur conditionné pour la compression du flux de gaz de synthèse brut ;
(C) une première colonne de lavage (1a) conditionnée pour le lavage du flux de gaz de synthèse brut avec du NH₃ liquide avec formation d'un premier condensat enrichi en CO₂ et d'un flux de gaz de synthèse prépurifié appauvri en CO₂ ;
(D) un échangeur de chaleur (13) conditionné pour refroidir le flux de gaz de synthèse prépurifié et un séparateur (22) conditionné pour la séparation d'une première phase liquide ;
(E) une deuxième colonne de lavage (1b) conditionnée pour le lavage du flux de gaz de synthèse prépurifié avec du NH₃ liquide avec formation d'un deuxième condensat enrichi en CO₂ et d'un flux de gaz de synthèse purifié appauvri en CO₂ ;
(F) un échangeur de chaleur (14) conditionné pour refroidir le flux de gaz de synthèse purifié et un séparateur (23) conditionné pour la séparation d'une deuxième phase liquide ;
(G) une troisième colonne de lavage (1c) conditionnée pour le lavage du flux de gaz de synthèse purifié avec du NH₃ liquide avec formation d'un troisième condensat enrichi en CO₂ et d'un flux de gaz de synthèse post-purifié appauvri en CO₂ ;
(H) un échangeur de chaleur (15) conditionné pour refroidir le flux de gaz de synthèse post-purifié et un séparateur (24) conditionné pour la séparation d'une troisième phase liquide ;
(I) des moyens pour la réunion du premier condensat de (C), du deuxième condensat de (E) et du troisième condensat de (G) avec formation d'un flux d'éduit ; et
(J) un réacteur à urée (2) conditionné pour la synthèse d'urée à partir du flux d'éduit avec formation d'une composition aqueuse d'urée et d'un flux de gaz résiduel comprenant NH₃ et CO₂ n'ayant pas réagi ;
le dispositif étant agencé pour la division du flux de gaz de synthèse brut du compresseur (5) en un premier flux partiel de gaz de synthèse brut et un deuxième flux partiel de gaz de synthèse brut ; et étant agencé pour le strippage de la composition aqueuse d'urée formée avec le premier flux partiel de gaz de synthèse brut avec formation d'un flux de gaz strippé ;
et étant agencé pour la réunion du flux de gaz strippé avec le deuxième flux partiel de gaz de synthèse brut; et
étant agencé pour le refroidissement du flux de gaz de synthèse brut ainsi réuni au moyen d'un échangeur de chaleur (12) et pour la séparation d'une quatrième phase liquide au moyen d'un séparateur (21).

14. Dispositif selon la revendication précédente, le dispositif conditionné pour la mise à disposition d'un flux de gaz de synthèse brut comprenant un reformeur à vapeur et/ou un reformeur autotherme ; et/ou le dispositif ne comprenant aucun compresseur conditionné pour la compression de CO₂ pur; et/ou le dispositif comprenant un réacteur d'ammoniac conditionné pour la synthèse d'ammoniac.

15. Utilisation d'un dispositif selon l'une quelconque des revendications de dispositif précédentes dans un procédé selon l'une quelconque des revendications de procédé précédentes.
